# EUROPÄISCHE PATENTSCHRIFT

(11) **EP 1 695 085 B1**
(45) Veröffentlichungstag und Bekanntmachung des Hinweises auf die Patenterteilung: **26.08.2009**
(21) Anmeldenummer: 04802964.9
(22) Anmeldetag: 20.12.2004
(51) Int. Cl.: G01N 33/569, G01N 33/92

(54) **VERFAHREN ZUM NACHWEIS VON ENDOTOXIN**
ENDOTOXIN DETECTION METHOD
PROCEDE DE DECELEMENT D'ENDOTOXINES

(30) Priorität: 20.12.2003 DE 10360844
(43) Veröffentlichungstag der Anmeldung: 30.08.2006
(73) Patentinhaber: Hyglos Invest GmbH, 82347 Bernried (DE)
(72) Erfinder: MEYER, Roman, 92287 Schmidmühlen (DE); SCHÜTZ, Michael, 93138 Kareth-Lappersdorf (DE); GRALLERT, Holger, 69198 Schriesheim (DE); GRASSL, Renate, 93049 Regensburg (DE); MILLER, Stefan, 93053 Regensburg (DE)
(74) Vertreter: Dehmel, Albrecht
(86) Internationale Anmeldenummer: PCT/DE2004/002778
(87) Internationale Veröffentlichungsnummer: WO 2005/062051

(56) Entgegenhaltungen:
- WO-A-03/000888
- WO-A-20/04001418
- US-B1- 6 436 653
- THOMASSEN E ET AL: "The Structure of the Receptor-binding Domain of the Bacteriophage T4 Short Tail Fibre Reveals a Knitted Trimeric Metal-binding Fold" JOURNAL OF MOLECULAR BIOLOGY, LONDON, GB, Bd. 331, Nr. 2, 8. August 2003 (2003-08-08), Seiten 361-373, XP004441503 ISSN: 0022-2836
- BAXA ULRICH ET AL: "Enthalpic barriers to the hydrophobic binding of oligosaccharides to phage P22 tailspike protein" BIOCHEMISTRY, Bd. 40, Nr. 17, 1. Mai 2001 (2001-05-01), Seiten 5144-5150, XP002330689 ISSN: 0006-2960
- SUN W ET AL: "USE OF BIOLUMINESCENT SALMONELLA FOR ASSESSING THE EFFICIENCY OF CONSTRUCTED PHAGE-BASED BIOSORBENT" JOURNAL OF INDUSTRIAL MICROBIOLOGY AND BIOTECHNOLOGY, BASINGSTOKE, GB, Bd. 25, Nr. 5, November 2000 (2000-11), Seiten 273-275, XP008016601 ISSN: 1367-5435
- SUZUKI ET AL.: "Specific interaction of fused H protein of bacteriophage lambdaX174 with receptor lipopolysaccharides" VIRUS RESEARCH, Bd. 60, 1999, Seiten 95-99,

### SEQUENCE LISTING

<110> PROFOS AG
<120> Verfahren zum Nachweis von Endotoxin
<130> PRO-013 PCT
<140> unknown
   <141> 2004-12-20
<150> DE 103 60 844.3
   <151> 2003-12-20
<160> 15
<170> PatentIn version 3.1
<210> 1
   <211> 78
   <212> DNA
   <213> artificial sequence
<220>
   <223> primer
<400> 1
   gaaggaacta gtcatatggc tagctggagc cacccgcagt tcgaaaaagg cgccagtaat 60
   aatacatatc aacacgtt 78
<210> 2
   <211> 54
   <212> DNA
   <213> artificial sequence
<220>
   <223> primer
<400> 2
   acgcgcaaag cttgtcgacg gatcctatca ttcttttacc ttaattatgt agtt 54
<210> 3
   <211> 78
   <212> DNA
   <213> artificial sequence
<220>
   <223> primer
<400> 3
   gaaggaacta gtcatatggc ttgttggagc cacccgcagt tcgaaaaagg cgccagtaat 60
   aatacatatc aacacgtt 78
<210> 4
   <211> 78
   <212> DNA
   <213> artificial sequence
<220>
   <223> primer
<400> 4
   gaaggaacta gtcatatggc tagctggagc cacccgcagt tcgaaaaagg cgcctgtaat 60
   aatacatatc aacacgtt 78
<210> 5
   <211> 19
   <212> PRT
   <213> artificial sequence
<220>
   <223> strep tag
<400> 5
   Met Ala Ser Trp Ser His Pro Gln Phe Glu Lys Gly Ala Ser Asn Asn
   1               5                   10                  15
   Thr Tyr Gln
<210> 6
   <211> 19
   <212> PRT
   <213> artificial sequence
<220>
   <223> strep tag
<400> 6
   Met Ala Cys Trp Ser His Pro Gln Phe Glu Lys Gly Ala Ser Asn Asn
   1               5                   10                  15
   Thr Tyr Gln
<210> 7
   <211> 19
   <212> PRT
   <213> artificial sequence
<220>
   <223> strep tag
<400> 7
   Met Ala Ser Trp Ser His Pro Gln Phe Glu Lys Gly Ala Cys Asn Asn
   1               5                   10                  15
   Thr Tyr Gln
<210> 8
   <211> 539
   <212> PRT
   <213> artificial sequence
<220>
   <223> T4p12 with strep tag
<400> 8
   Met Ala Ser Trp Ser His Pro Gln Phe Glu Lys Gly Ala Ser Asn Asn
   1               5                   10                  15
   Thr Tyr Gln His Val Ser Asn Glu Ser Arg Tyr Val Lys Phe Asp Pro
               20                  25                  30
   Thr Asp Thr Asn Phe Pro Pro Glu Ile Thr Asp Val Gln Ala Ala Ile
           35                  40                  45
   Ala Ala Ile Ser Pro Ala Gly Val Asn Gly Val Pro Asp Ala Ser Ser
       50                  55                  60
   Thr Thr Lys Gly Ile Leu Phe Leu Ala Thr Glu Gln Glu Val Ile Asp
   65                  70                  75                  80
   Gly Thr Asn Asn Thr Lys Ala Val Thr Pro Ala Thr Leu Ala Thr Arg
                   85                  90                  95
   Leu Ser Tyr Pro Asn Ala Thr Glu Ala Val Tyr Gly Leu Thr Arg Tyr
               100                 105                 110
   Ser Thr Asp Asp Glu Ala Ile Ala Gly Val Asn Asn Glu Ser Ser Ile
           115                 120                 125
   Thr Pro Ala Lys Phe Thr Val Ala Leu Asn Asn Val Phe Glu Thr Arg
       130                 135                 140
   Val Ser Thr Glu Ser Ser Asn Gly Val Ile Lys Ile Ser Ser Leu Pro
   145                 150                 155                 160
   Gln Ala Leu Ala Gly Ala Asp Asp Thr Thr Ala Met Thr Pro Leu Lys
                   165                 170                 175
   Thr Gln Gln Leu Ala Val Lys Leu Ile Ala Gln Ile Ala Pro Ser Lys
               180                 185                 190
   Asn Ala Ala Thr Glu Ser Glu Gln Gly Val Ile Gln Leu Ala Thr Val
           195                 200                 205
   Ala Gln Ala Arg Gln Gly Thr Leu Arg Glu Gly Tyr Ala Ile Ser Pro
       210                 215                 220
   Tyr Thr Phe Met Asn Ser Thr Ala Thr Glu Glu Tyr Lys Gly Val Ile
   225                 230                 235                 240
   Lys Leu Gly Thr Gln Ser Glu Val Asn Ser Asn Asn Ala Ser Val Ala
                   245                 250                 255
   Val Thr Gly Ala Thr Leu Asn Gly Arg Gly Ser Thr Thr Ser Met Arg
               260                 265                 270
   Gly Val Val Lys Leu Thr Thr Thr Ala Gly Ser Gln Ser Gly Gly Asp
           275                 280                 285
   Ala Ser Ser Ala Leu Ala Trp Asn Ala Asp Val Ile His Gln Arg Gly
       290                 295                 300
   Gly Gln Thr Ile Asn Gly Thr Leu Arg Ile Asn Asn Thr Leu Thr Ile
   305                 310                 315                 320
   Ala Ser Gly Gly Ala Asn Ile Thr Gly Thr Val Asn Met Thr Gly Gly
   325 330 335
   Tyr Ile Gln Gly Lys Arg Val Val Thr Gln Asn Glu Ile Asp Arg Thr
   340 345 350
   Ile Pro Val Gly Ala Ile Met Met Trp Ala Ala Asp Ser Leu Pro Ser
   355 360 365
   Asp Ala Trp Arg Phe Cys His Gly Gly Thr Val Ser Ala Ser Asp Cys
   370 375 380
   Pro Leu Tyr Ala Ser Arg Ile Gly Thr Arg Tyr Gly Gly Ser Ser Ser
   385 390 395 400
   Asn Pro Gly Leu Pro Asp Met Arg Gly Leu Phe Val Arg Gly Ser Gly
   405 410 415
   Arg Gly Ser His Leu Thr Asn Pro Asn Val Asn Gly Asn Asp Gln Phe
   420 425 430
   Gly Lys Pro Arg Leu Gly Val Gly Cys Thr Gly Gly Tyr Val Gly Glu
   435 440 445
   Val Gln Lys Gln Gln Met Ser Tyr His Lys His Ala Gly Gly Phe Gly
   450 455 460
   Glu Tyr Asp Asp Ser Gly Ala Phe Gly Asn Thr Arg Arg Ser Asn Phe
   465 470 475 480
   Val Gly Thr Arg Lys Gly Leu Asp Trp Asp Asn Arg Ser Tyr Phe Thr
   485 490 495
   Asn Asp Gly Tyr Glu Ile Asp Pro Ala Ser Gln Arg Asn Ser Arg Tyr
   500 505 510
   Thr Leu Asn Arg Pro Glu Leu Ile Gly Asn Glu Thr Arg Pro Trp Asn
   515 520 525
   Ile Ser Leu Asn Tyr Ile Ile Lys Val Lys Glu
   530 535
<210> 9
   <211> 527
   <212> PRT
   <213> protein p12 of T2 phage
<400> 9
   Met Ser Asn Asn Thr Tyr Gln His Val Ser Asn Glu Ser Arg Tyr Val
   1               5                   10                  15
   Lys Phe Asp Pro Thr Asp Thr Asn Phe Pro Pro Glu Ile Thr Asp Val
               20                  25                  30
   Gln Ala Ala Ile Ala Ala Ile Ser Pro Ala Gly Val Asn Gly Val Pro
           35                  40                  45
   Asp Ala Ser Ser Thr Thr Lys Gly Ile Leu Phe Leu Ala Thr Glu Gln
       50                  55                  60
   Glu Val Ile Asp Gly Thr Asn Asn Thr Lys Ala Val Thr Pro Ala Thr
   65                  70                  75                  80
   Leu Ala Thr Arg Leu Ser Tyr Pro Asn Ala Thr Glu Ala Val Tyr Gly
                   85                  90                  95
   Leu Thr Arg Tyr Ser Thr Asp Asp Glu Ala Ile Ala Gly Val Asn Asn
               100                 105                 110
   Glu Ser Ser Ile Thr Pro Ala Lys Phe Thr Val Ala Leu Asn Asn Val
           115                 120                 125
   Phe Glu Thr Arg Val Ser Thr Glu Ser Ser Asn Gly Val Ile Lys Ile
       130                 135                 140
   Ser Ser Leu Pro Gln Ala Leu Ala Gly Ala Asp Asp Thr Thr Ala Met
   145                 150                 155                 160
   Thr Pro Leu Lys Thr Gln Gln Leu Ala Val Lys Leu Ile Ala Gln Ile
                   165                 170                 175
   Ala Pro Ser Lys Asn Ala Ala Thr Glu Ser Glu Gln Gly Val Ile Gln
               180                 185                 190
   Leu Ala Thr Val Ala Gln Ala Arg Gln Gly Thr Leu Arg Glu Gly Tyr
           195                 200                 205
   Ala Ile Ser Pro Tyr Thr Phe Met Asn Ser Thr Ala Thr Glu Glu Tyr
       210                 215                 220
   Lys Gly Val Ile Lys Leu Gly Thr Gln Ser Glu Val Asn Ser Asn Asn
   225                 230                 235                 240
   Ala Ser Val Ala Val Thr Gly Ala Thr Leu Asn Gly Arg Gly Ser Thr
                   245                 250                 255
   Thr Ser Met Arg Gly Val Val Lys Leu Thr Thr Thr Ala Gly Ser Gln
               260                 265                 270
   Ser Gly Gly Asp Ala Ser Ser Ala Leu Ala Trp Asn Ala Asp Val Ile
           275                 280                 285
   His Gln Arg Gly Gly Gln Thr Ile Asn Gly Thr Leu Arg Ile Asn Asn
       290                 295                 300
   Thr Leu Thr Ile Ala Ser Gly Gly Ala Asn Ile Thr Gly Thr Val Asn
   305                 310                 315                 320
   Met Thr Gly Gly Tyr Ile Gln Gly Lys Arg Val Val Thr Gln Asn Glu
                   325                 330                 335
   Ile Asp Arg Thr Ile Pro Val Gly Ala Ile Met Met Trp Ala Ala Asp
               340                 345                 350
   Ser Leu Pro Ser Asp Ala Trp Arg Phe Cys His Gly Gly Thr Val Ser
           355                 360                 365
   Ala Ser Asp Cys Pro Leu Tyr Ala Ser Arg Ile Gly Thr Arg Tyr Gly
       370                 375                 380
   Gly Thr Ser Ser Asn Pro Gly Leu Pro Asp Met Arg Gly Leu Phe Val
   385                 390                 395                 400
   Arg Gly Ser Gly Arg Gly Ser His Leu Thr Asn Pro Asn Val Asn Gly
                   405                 410                 415
   Asn Asp Gln Phe Gly Lys Pro Arg Leu Gly Val Gly Cys Thr Gly Gly
               420                 425                 430
   Tyr Val Gly Glu Val Gln Lys Gln Gln Met Ser Tyr His Lys His Ala
           435                 440                 445
   Gly Gly Phe Gly Glu Tyr Asp Asp Ser Gly Ala Phe Gly Asn Thr Arg
       450                 455                 460
   Arg Ser Asn Phe Val Gly Thr Arg Lys Gly Leu Asp Trp Asp Asn Arg
   465                 470                 475                 480
   Ser Tyr Phe Thr Asn Asp Gly Tyr Glu Ile Asp Pro Ala Ser Gln Arg
                   485                 490                 495
   Asn Ser Arg Tyr Thr Leu Asn Arg Pro Glu Leu Ile Gly Asn Glu Thr
               500                 505                 510
   Arg Pro Trp Asn Ile Ser Leu Asn Tyr Ile Ile Lys Val Lys Glu
           515                 520                 525
<210> 10
   <211> 527
   <212> PRT
   <213> protein p12 of T4 phage
<400> 10
   Met Ser Asn Asn Thr Tyr Gln His Val Ser Asn Glu Ser Arg Tyr Val
   1               5                   10                  15
   Lys Phe Asp Pro Thr Asp Thr Asn Phe Pro Pro Glu Ile Thr Asp Val
               20                  25                  30
   His Ala Ala Ile Ala Ala Ile Ser Pro Ala Gly Val Asn Gly Val Pro
           35                  40                  45
   Asp Ala Ser Ser Thr Thr Lys Gly Ile Leu Phe Ile Pro Thr Glu Gln
       50                  55                  60
   Glu Val Ile Asp Gly Thr Asn Asn Thr Lys Ala Val Thr Pro Ala Thr
   65                  70                  75                  80
   Leu Ala Thr Arg Leu Ser Tyr Pro Asn Ala Thr Glu Thr Val Tyr Gly
                   85                  90                  95
   Leu Thr Arg Tyr Ser Thr Asn Asp Glu Ala Ile Ala Gly Val Asn Asn
               100                 105                 110
   Glu Ser Ser Ile Thr Pro Ala Lys Phe Thr Val Ala Leu Asn Asn Ala
           115                 120                 125
   Phe Glu Thr Arg Val Ser Thr Glu Ser Ser Asn Gly Val Ile Lys Ile
       130                 135                 140
   Ser Ser Leu Pro Gln Ala Leu Ala Gly Ala Asp Asp Thr Thr Ala Met
   145                 150                 155                 160
   Thr Pro Leu Lys Thr Gln Gln Leu Ala Ile Lys Leu Ile Ala Gln Ile
                   165                 170                 175
   Ala Pro Ser Glu Thr Thr Ala Thr Glu Ser Asp Gln Gly Val Val Gln
   180 185 190
   Leu Ala Thr Val Ala Gln Val Arg Gln Gly Thr Leu Arg Glu Gly Tyr
   195 200 205
   Ala Ile Ser Pro Tyr Thr Phe Met Asn Ser Ser Ser Thr Glu Glu Tyr
   210 215 220
   Lys Gly Val Ile Lys Leu Gly Thr Gln Ser Glu Val Asn Ser Asn Asn
   225 230 235 240
   Ala Ser Val Ala Val Thr Gly Ala Thr Leu Asn Gly Arg Gly Ser Thr
   245 250 255
   Thr Ser Met Arg Gly Val Val Lys Leu Thr Thr Thr Ala Gly Ser Gln
   260 265 270
   Ser Gly Gly Asp Ala Ser Ser Ala Leu Ala Trp Asn Ala Asp Val Ile
   275 280 285
   Gln Gln Arg Gly Gly Gln Ile Ile Tyr Gly Thr Leu Arg Ile Glu Asp
   290 295 300
   Thr Phe Thr Ile Ala Asn Gly Gly Ala Asn Ile Thr Gly Thr Val Arg
   305 310 315 320
   Met Thr Gly Gly Tyr Ile Gln Gly Asn Arg Ile Val Thr Gln Asn Glu
   325 330 335
   Ile Asp Arg Thr Ile Pro Val Gly Ala Ile Met Met Trp Ala Ala Asp
   340 345 350
   Ser Leu Pro Ser Asp Ala Trp Arg Phe Cys His Gly Gly Thr Val Ser
   355 360 365
   Ala Ser Asp Cys Pro Leu Tyr Ala Ser Arg Ile Gly Thr Arg Tyr Gly
   370 375 380
   Gly Asn Pro Ser Asn Pro Gly Leu Pro Asp Met Arg Gly Leu Phe Val
   385 390 395 400
   Arg Gly Ser Gly Arg Gly Ser His Leu Thr Asn Pro Asn Val Asn Gly
   405 410 415
   Asn Asp Gln Phe Gly Lys Pro Arg Leu Gly Val Gly Cys Thr Gly Gly
   420 425 430
   Tyr Val Gly Glu Val Gln Ile Gln Gln Met Ser Tyr His Lys His Ala
   435 440 445
   Gly Gly Phe Gly Glu His Asp Asp Leu Gly Ala Phe Gly Asn Thr Arg
   450 455 460
   Arg Ser Asn Phe Val Gly Thr Arg Lys Gly Leu Asp Trp Asp Asn Arg
   465 470 475 480
   Ser Tyr Phe Thr Asn Asp Gly Tyr Glu Ile Asp Pro Glu Ser Gln Arg
   485 490 495
   Asn Ser Lys Tyr Thr Leu Asn Arg Pro Glu Leu Ile Gly Asn Glu Thr
   500 505 510
   Arg Pro Trp Asn Ile Ser Leu Asn Tyr Ile Ile Lys Val Lys Glu
   515 520 525
<210> 11
   <211> 518
   <212> PRT
   <213> protein p12 of PP01 phage
<400> 11
   Met Ser Asn Asn Thr Tyr Gln His Val Ser Asn Glu Ser Lys Tyr Val
   1               5                   10                  15
   Lys Phe Asp Pro Val Gly Ser Asn Phe Pro Asp Thr Val Thr Thr Val
               20                  25                  30
   Gln Ser Ala Leu Ser Lys Ile Ser Asn Ile Gly Val Asn Gly Ile Pro
           35                  40                  45
   Asp Ala Ser Met Glu Val Lys Gly Ile Ala Met Ile Ala Ser Glu Gln
       50                  55                  60
   Glu Val Leu Asp Gly Thr Asn Asn Ser Lys Ile Val Thr Pro Ala Thr
   65                  70                  75                  80
   Leu Ala Thr Arg Leu Leu Tyr Pro Asn Ala Thr Glu Thr Lys Tyr Gly
                   85                  90                  95
   Leu Thr Arg Tyr Ser Thr Asn Glu Glu Thr Leu Glu Gly Ser Asp Asn
               100                 105                 110
   Asn Ser Ser Ile Thr Pro Gln Lys Leu Lys Tyr His Thr Asp Asp Val
           115                 120                 125
   Phe Gln Asn Arg Tyr Ser Ser Glu Ser Ser Asn Gly Val Ile Lys Ile
       130                 135                 140
   Ser Ser Thr Pro Ala Ala Leu Ala Gly Val Asp Asp Thr Thr Ala Met
   145                 150                 155                 160
   Thr Pro Leu Lys Thr Gln Lys Leu Ala Ile Lys Leu Ile Ser Gln Ile
                   165                 170                 175
   Ala Pro Ser Glu Asp Thr Ala Ser Glu Ser Val Arg Gly Val Val Gln
               180                 185                 190
   Leu Ser Thr Val Ala Gln Thr Arg Gln Gly Thr Leu Arg Glu Gly Tyr
           195                 200                 205
   Ala Ile Ser Pro Tyr Thr Phe Met Asn Ser Val Ala Thr Gln Glu Tyr
       210                 215                 220
   Lys Gly Val Ile Arg Leu Gly Thr Gln Ser Glu Ile Asn Ser Asn Leu
   225                 230                 235                 240
   Gly Asp Val Ala Val Thr Gly Glu Thr Leu Asn Gly Arg Gly Ala Thr
                   245                 250                 255
   Gly Ser Met Arg Gly Val Val Lys Leu Thr Thr Gln Ala Gly Ile Ala
               260                 265                 270
   Pro Glu Gly Asp Ser Ser Gly Ala Leu Ala Trp Asn Ala Asp Val Ile
           275                 280                 285
   Asn Thr Arg Gly Gly Gln Thr Ile Asn Gly Ser Leu Asn Leu Asp His
   290                 295                 300
   Leu Thr Ala Asn Gly Ile Trp Ser Arg Gly Gly Met Trp Lys Asn Gly
   305                 310                 315                 320
   Asp Gln Pro Val Ala Thr Glu Arg Tyr Ala Ser Glu Arg Val Pro Val
                   325                 330                 335
   Gly Thr Ile Met Met Phe Ala Gly Asp Ser Ala Pro Pro Gly Trp Ile
               340                 345                 350
   Met Cys His Gly Gly Thr Val Ser Gly Asp Gln Tyr Pro Asp Tyr Arg
           355                 360                 365
   Asn Thr Val Gly Thr Arg Phe Gly Gly Asp Trp Asn Asn Pro Gly Ile
       370                 375                 380
   Pro Asp Met Arg Gly Leu Phe Val Arg Gly Ala Gly Thr Gly Gly His
   385                 390                 395                 400
   Ile Leu Asn Gln Arg Gly Gln Asp Gly Tyr Gly Lys Asp Arg Leu Gly
                   405                 410                 415
   Val Gly Cys Asp Gly Met His Val Gly Gly Val Gln Ala Gln Gln Ile
               420                 425                 430
   Ser Tyr His Lys His Ala Gly Ala Trp Gly Glu Asn Gly Asn Asn Arg
           435                 440                 445
   Gly Tyr Ala Pro Phe Gly Ala Ser Asn Gly Ser Gly Tyr Leu Gly Asn
       450                 455                 460
   Gly Arg Ser Ala Asp Trp Asp Asn His Leu Phe Phe Thr Asn Asp Gly
   465                 470                 475                 480
   Phe Glu Met Gly Gly Pro Arg Asp Ser Phe Gly Thr Leu Asn Arg Glu
                   485                 490                 495
   Gly Leu Ile Gly Tyr Glu Thr Arg Pro Trp Asn Ile Ser Leu Asn Tyr
               500                 505                 510
   Ile Ile Lys Ile His Tyr
           515
<210> 12
   <211> 516
   <212> PRT
   <213> protein p12 of RB69 phage
<400> 12
   Met Ser Asn Asn Thr Tyr Gln His Val Ser Asn Glu Ser Val Tyr Val
   1 5 10 15
   Glu Phe Asp Pro Thr Gly Ser Asn Phe Asp Ser Ser Ile Thr Asn Val
   20 25 30
   Gln Ala Ala Leu Ala Ser Ile Ser Ala Tyr Gly Val Lys Gly Val Pro
   35 40 45
   Asp Ala Ser Glu Ala Glu Lys Gly Val Ile Gln Leu Ala Thr Glu Gln
   50 55 60
   Glu Val Leu Asp Gly Phe Asn Ser Thr Lys Ala Val Thr Pro Ala Thr
   65                  70                  75                  80
   Leu Asn Ala Arg Leu Gln Tyr Pro Asn Ala Ser Glu Thr Gln Tyr Gly
                   85                  90                  95
   Val Thr Lys Tyr Ala Thr Gln Glu Glu Ala Ile Ala Gly Thr Leu Asp
               100                 105                 110
   Thr Val Ser Ile Thr Pro Leu Lys Leu Asn Gln Thr Ile Asp Asn Thr
           115                 120                 125
   Phe Ser Thr Arg Tyr Ser Thr Glu Thr Thr Asn Gly Val Ile Lys Ile
       130                 135                 140
   Ala Thr Gln Thr Ala Ala Leu Ala Gly Ser Asp Asp Thr Thr Ala Met
   145                 150                 155                 160
   Thr Pro Leu Lys Thr Gln Gln Leu Ala Ile Lys Leu Ile Ser Gln Ile
                   165                 170                 175
   Ala Pro Asn Asn Asp Pro Ala Ser Glu Ser Ile Thr Gly Val Val Arg
               180                 185                 190
   Leu Ala Thr Val Ala Gln Thr Arg Gln Gly Thr Leu Arg Glu Gly Tyr
           195                 200                 205
   Ala Ile Ser Pro Tyr Thr Phe Met Asn Ser Val Ala Thr Gln Glu Tyr
       210                 215                 220
   Lys Gly Val Ile Arg Leu Gly Thr Gln Ala Glu Ile Asn Ser Asn Leu
   225                 230                 235                 240
   Gly Asp Val Ala Val Thr Gly Glu Thr Leu Asn Gly Arg Gly Ala Thr
                   245                 250                 255
   Gly Ser Met Arg Gly Val Val Lys Leu Thr Thr Gln Ala Gly Val Ala
               260                 265                 270
   Pro Glu Gly Asp Ser Ser Gly Ala Leu Ala Trp Asn Ala Asp Val Ile
           275                 280                 285
   Asn Thr Arg Gly Gly Gln Thr Ile Asn Gly Ser Leu Asn Leu Asp His
       290                 295                 300
   Leu Thr Ala Asn Gly Ile Trp Ser Arg Gly Gly Met Trp Lys Asn Gly
   305                 310                 315                 320
   Asp Gln Pro Val Ala Thr Glu Arg Tyr Ala Ser Glu Arg Val Pro Val
                   325                 330                 335
   Gly Thr Ile Gln Met Phe Ala Gly Asp Ser Ala Pro Pro Gly Trp Val
               340                 345                 350
   Leu Cys His Gly Gly Thr Ile Ser Gly Asp Gln Phe Pro Asp Tyr Arg
           355                 360                 365
   Asn Val Val Gly Thr Arg Phe Gly Gly Asp Trp Asn Asn Pro Gly Ile
       370                 375                 380
   Pro Asp Met Arg Gly Leu Phe Val Arg Gly Ala Gly Thr Gly Ser His
   385                 390                 395                 400
   Ile Leu Asn Asn Arg Gly Gln Asp Gly Tyr Gly Lys Asp Arg Leu Gly
                   405                     410                     415
   Val Gly Cys Asp Gly Met His Val Gly Gly Val Gln Ala Gln Gln Met
               420                     425                     430
   Ser Tyr His Lys His Ala Gly Gly Trp Gly Glu Phe Gln Arg His Glu
           435                     440                     445
   Ala Pro Phe Gly Ala Ser Val Tyr Gln Gly Tyr Leu Gly Thr Arg Lys
       450                     455                     460
   Tyr Ser Asp Trp Asp Asn Ala Ser Tyr Phe Thr Asn Asp Gly Phe Glu
   465                     470                     475                     480
   Leu Gly Gly His Arg Asp Ala Thr Gly Thr Leu Asn Arg Glu Gly Leu
                   485                     490                     495
   Ile Gly Tyr Glu Thr Arg Pro Trp Asn Ile Ser Leu Asn Tyr Ile Ile
               500                     505                     510
   Lys Val His Tyr
           515
<210> 13
   <211> 516
   <212> PRT
   <213> protein p12 of AR1 phage
<400> 13
   Met Ser Asn Asn Thr Tyr Gln His Val Ser Asn Glu Ser Lys Tyr Val
   1               5                   10                  15
   Lys Phe Asp Pro Thr Gly Ser Asn Phe Pro Asp Thr Val Thr Thr Val
               20                  25                  30
   Gln Ser Ala Leu Ser Lys Ile Ser Asn Ile Gly Val Asn Gly Ile Pro
           35                  40                  45
   Asp Ala Thr Met Glu Val Lys Gly Ile Ala Met Ile Ala Ser Glu Gln
       50                  55                  60
   Glu Val Leu Asp Gly Thr Asn Asn Ser Lys Ile Val Thr Pro Ala Thr
   65                  70                  75                  80
   Leu Ala Thr Arg Leu Leu Tyr Pro Asn Ala Thr Glu Thr Lys Tyr Gly
                   85                  90                  95
   Leu Thr Arg Tyr Ser Thr Asn Glu Glu Thr Leu Glu Gly Ser Asp Asn
               100                 105                 110
   Asn Ser Ser Ile Thr Pro Gln Lys Leu Lys Tyr His Thr Asp Asp Val
           115                 120                 125
   Phe Gln Asn Arg Tyr Ser Ser Glu Ser Ser Asn Gly Val Ile Lys Ile
       130                 135                 140
   Ser Ser Thr Pro Ala Ala Leu Ala Gly Val Asp Asp Thr Thr Ala Met
   145                 150                 155                 160
   Thr Pro Leu Lys Thr Gln Lys Leu Ala Ile Lys Leu Ile Ser Gln Ile
                   165                 170                 175
   Ala Pro Ser Glu Asp Thr Ala Ser Glu Ser Val Arg Gly Val Val Gln
               180                 185                 190
   Leu Ser Thr Val Ala Gln Thr Arg Gln Gly Thr Leu Arg Glu Gly Tyr
           195                 200                 205
   Ala Ile Ser Pro Tyr Thr Phe Met Asn Ser Val Ala Thr Gln Glu Tyr
       210                 215                 220
   Lys Gly Val Ile Arg Leu Gly Thr Gln Ser Glu Ile Asn Ser Asn Leu
   225                 230                 235                 240
   Gly Asp Val Ala Val Thr Gly Gly Thr Leu Asn Gly Arg Gly Ala Thr
                   245                 250                 255
   Gly Ser Met Arg Gly Val Val Lys Leu Thr Thr Gln Ala Gly Ile Ala
               260                 265                 270
   Pro Glu Gly Asp Ser Ser Gly Ala Leu Ala Trp Asn Ala Asp Val Ile
           275                 280                 285
   Asn Thr Arg Gly Gly Gln Thr Ile Asn Gly Ser Leu Asn Leu Asp His
       290                 295                 300
   Leu Thr Ala Asn Gly Ile Trp Ser Arg Gly Gly Met Trp Lys Asn Gly
   305                 310                 315                 320
   Asp Gln Pro Val Ala Thr Glu Arg Tyr Ala Ser Glu Arg Val Pro Val
                   325                 330                 335
   Gly Thr Ile Met Met Phe Ala Gly Asp Ser Ala Pro Pro Gly Trp Ile
               340                 345                 350
   Met Cys His Gly Gly Thr Val Ser Gly Asp Gln Tyr Pro Asp Tyr Arg
           355                 360                 365
   Asn Thr Val Gly Thr Arg Phe Gly Gly Asp Trp Asn Asn Pro Gly Ile
       370                 375                 380
   Pro Asp Met Arg Gly Leu Phe Val Arg Gly Ala Gly Thr Gly Gly His
   385                 390                 395                 400
   Ile Leu Asn Gln Arg Gly Gln Asp Gly Tyr Gly Lys Asp Arg Leu Gly
                   405                 410                 415
   Val Gly Cys Asp Gly Met His Val Gly Gly Val Gln Ala Gln Gln Met
               420                 425                 430
   Ser Tyr His Lys His Ala Gly Gly Trp Gly Glu Tyr Asn Arg Ser Glu
           435                 440                 445
   Gly Pro Phe Gly Ala Ser Val Tyr Gln Gly Tyr Leu Gly Thr Arg Lys
       450                 455                 460
   Tyr Ser Asp Trp Asp Asn Ala Ser Tyr Phe Thr Asn Asp Gly Phe Glu
   465                 470                 475                 480
   Leu Gly Gly Pro Arg Asp Ala Leu Gly Thr Leu Asn Arg Glu Gly Leu
                   485                 490                 495
   Ile Gly Tyr Glu Thr Arg Pro Trp Asn Ile Ser Leu Asn Tyr Ile Ile
               500                 505                 510
   Lys Ile His Tyr
   515
<210> 14
   <211> 527
   <212> PRT
   <213> protein p12 of K3 phage
<400> 14
   Met Ser Asn Asn Thr Tyr Gln His Val Ser Asn Glu Ser Arg Tyr Val
   1               5                   10                  15
   Lys Phe Asp Pro Thr Asp Thr Asn Phe Pro Pro Glu Ile Thr Asp Val
               20                  25                  30
   Gln Ala Ala Ile Ala Ala Ile Ser Pro Ala Gly Val Asn Gly Val Pro
           35                  40                  45
   Asp Ala Ser Ser Thr Thr Lys Gly Ile Leu Phe Leu Ala Thr Glu Gln
       50                  55                  60
   Glu Val Ile Asp Gly Thr Asn Asn Thr Lys Ala Val Thr Pro Ala Thr
   65                  70                  75                  80
   Leu Ala Thr Arg Leu Ser Tyr Pro Asn Ala Thr Glu Thr Val Tyr Gly
                   85                  90                  95
   Leu Thr Arg Tyr Ser Thr Asn Asp Glu Ala Ile Ala Gly Val Asn Asn
               100                 105                 110
   Glu Ser Ser Ile Thr Pro Ala Lys Phe Thr Val Ala Leu Asn Asn Ala
           115                 120                 125
   Phe Glu Thr Arg Val Ser Thr Glu Ser Ser Asn Gly Val Ile Lys Ile
       130                 135                 140
   Ser Ser Leu Pro Gln Ala Leu Ala Gly Ala Asp Asp Thr Thr Ala Met
   145                 150                 155                 160
   Thr Pro Leu Lys Thr Gln Gln Leu Ala Ile Lys Leu Ile Ala Gln Ile
                   165                 170                 175
   Ala Pro Ser Glu Thr Thr Ala Thr Glu Ser Asp Gln Gly Val Val Gln
               180                 185                 190
   Leu Ala Thr Val Ala Gln Val Arg Gln Gly Thr Leu Arg Glu Gly Tyr
           195                 200                 205
   Ala Ile Ser Pro Tyr Thr Phe Met Asn Ser Ser Ala Thr Glu Glu Tyr
       210                 215                 220
   Lys Gly Val Ile Lys Leu Gly Thr Gln Ser Glu Val Asn Ser Asn Asn
   225                 230                 235                 240
   Ala Ser Val Ala Val Thr Gly Ala Thr Leu Asn Gly Arg Gly Ser Thr
                   245                 250                 255
   Thr Ser Met Arg Gly Val Val Arg Leu Thr Thr Thr Ala Gly Ser Gln
               260                 265                 270
   Ser Gly Gly Asp Ala Ser Ser Ala Leu Ala Trp Asn Ala Asp Val Ile
           275                 280                 285
   His Gln Arg Gly Gly Gln Thr Ile Asn Gly Thr Leu Arg Ile Asn Asn
       290                 295                 300
   Thr Leu Thr Ile Ala Ser Gly Gly Ala Asn Ile Thr Gly Thr Val Asn
   305                 310                 315                 320
   Met Thr Gly Gly Tyr Ile Gln Gly Lys Arg Val Val Thr Gln Asn Glu
                   325                 330                 335
   Ile Asp Arg Thr Ile Pro Val Gly Ala Ile Met Met Trp Ala Ala Asp
               340                 345                 350
   Ser Leu Pro Ser Asp Ala Trp Arg Phe Cys His Gly Gly Thr Val Ser
           355                 360                 365
   Ala Ser Asp Cys Pro Leu Tyr Ala Ser Arg Ile Gly Thr Arg Tyr Gly
       370                 375                 380
   Gly Ser Ser Ser Asn Pro Gly Leu Pro Asp Met Arg Gly Leu Phe Val
   385                 390                 395                 400
   Arg Gly Ser Gly Arg Gly Ser His Leu Thr Asn Pro Asn Val Asn Gly
                   405                 410                 415
   Asn Asp Gln Phe Gly Lys Pro Arg Leu Gly Val Gly Cys Thr Gly Gly
               420                 425                 430
   Tyr Val Gly Glu Val Gln Lys Gln Gln Met Ser Tyr His Lys His Ala
           435                 440                 445
   Gly Gly Phe Gly Glu Trp Asp Asp Ser Gly Ala Phe Gly Asn Thr Arg
       450                 455                 460
   Arg Ser Asn Phe Val Gly Thr Arg Lys Gly Leu Asp Trp Asp Asn Arg
   465                 470                 475                 480
   Ser Tyr Phe Thr Asn Asp Gly Tyr Glu Ile Asp Pro Ala Ser Gln Arg
                   485                 490                 495
   Asn Ser Arg Tyr Thr Leu Asn Arg Pro Glu Leu Ile Gly Asn Glu Thr
               500                 505                 510
   Arg Pro Trp Asn Ile Ser Leu Asn Tyr Ile Ile Lys Val Lys Glu
           515                 520                 525
<210> 15
   <211> 516
   <212> PRT
   <213> protein p12 of RB32-33 phage
<400> 15
   Met Ser Asn Asn Thr Tyr Gln His Val Ser Asn Glu Ser Lys Tyr Val
   1               5                   10                  15
   Lys Phe Asp Pro Val Gly Ser Asn Phe Pro Asp Thr Val Thr Thr Val
               20                  25                  30
   Gln Ser Ala Leu Ser Lys Ile Ser Asn Ile Gly Val Asn Gly Ile Pro
           35                  40                  45
   Asp Ala Thr Met Glu Val Lys Gly Ile Ala Met Ile Ala Ser Glu Gln
       50                  55                  60
   Glu Val Leu Asp Gly Thr Asn Asn Ser Lys Ile Val Thr Pro Ala Thr
   65 70 75 80
   Leu Ala Thr Arg Leu Leu Tyr Pro Asn Ala Thr Glu Thr Lys Tyr Gly
   85 90 95
   Leu Thr Arg Tyr Ser Thr Asn Glu Glu Thr Leu Glu Gly Ser Asp Asn
   100 105 110
   Asn Ser Ser Ile Thr Pro Gln Lys Leu Lys Tyr His Thr Asp Asp Val
   115 120 125
   Phe Gln Asn Arg Tyr Ser Ser Glu Ser Ser Asn Gly Val Ile Lys Ile
   130 135 140
   Ser Ser Thr Pro Ala Ala Leu Ala Gly Val Asp Asp Thr Thr Ala Met
   145 150 155 160
   Thr Pro Leu Lys Thr Gln Lys Leu Ala Ile Lys Leu Ile Ser Gln Ile
   165 170 175
   Ala Pro Ser Glu Asp Thr Ala Ser Glu Ser Val Arg Gly Val Val Gln
   180 185 190
   Leu Ser Thr Val Ala Gln Thr Arg Gln Gly Thr Leu Arg Glu Gly Tyr
   195 200 205
   Ala Ile Ser Pro Tyr Thr Phe Met Asn Ser Val Ala Thr Gln Glu Tyr
   210 215 220
   Lys Gly Val Ile Arg Leu Gly Thr Gln Ser Glu Ile Asn Ser Asn Leu
   225 230 235 240
   Gly Asp Val Ala Val Thr Gly Glu Thr Leu Asn Gly Arg Gly Ala Thr
   245 250 255
   Ser Ser Met Arg Gly Val Val Lys Leu Thr Thr Gln Ala Gly Ile Ala
   260 265 270
   Pro Glu Gly Asp Gly Ser Gly Ala Leu Ala Trp Asn Ala Asp Val Ile
   275 280 285
   Asn Thr Arg Gly Gly Gln Thr Ile Asn Gly Ser Leu Asn Leu Asp His
   290 295 300
   Leu Thr Ala Asn Gly Ile Trp Ser Arg Gly Gly Met Trp Lys Asn Gly
   305 310 315 320
   Asp Gln Pro Val Ala Thr Glu Arg Tyr Ala Ser Glu Arg Val Pro Val
   325 330 335
   Gly Thr Ile Met Met Phe Ala Gly Asp Ser Ala Pro Pro Gly Trp Ile
   340 345 350
   Met Cys His Gly Gly Thr Val Ser Gly Asp Gln Tyr Pro Asp Tyr Arg
   355 360 365
   Asn Thr Val Gly Ala Arg Phe Gly Gly Asp Trp Asn Asn Pro Gly Ile
   370 375 380
   Pro Asp Met Arg Gly Leu Phe Val Arg Gly Ala Gly Thr Gly Gly His
   385 390 395 400
   Ile Leu Asn Gln Arg Gly Gln Asp Gly Tyr Gly Lys Asp Arg Leu Gly
                   405                 410                 415
   Val Gly Cys Asp Gly Met His Val Gly Gly Val Gln Ala Gln Gln Met
               420                 425                 430
   Ser Tyr His Lys His Ala Gly Gly Trp Gly Glu Tyr Gln Arg His Glu
           435                 440                 445
   Ala Pro Phe Gly Ala Ser Val Tyr Gln Gly Tyr Leu Gly Thr Arg Lys
       450                 455                 460
   Tyr Ser Asp Trp Asp Asn Ala Ser Tyr Phe Thr Asn Asp Gly Phe Glu
   465                 470                 475                 480
   Leu Gly Gly Pro Arg Asp Ala Leu Gly Thr Leu Asn Arg Glu Gly Leu
                   485                 490                 495
   Ile Gly Tyr Glu Thr Arg Pro Trp Asn Ile Ser Leu Asn Tyr Ile Ile
               500                 505                 510
   Lys Ile His Tyr
           515

## Beschreibung

Die vorliegende Erfindung betrifft Verfahren zum Nachweis von Endotoxinen in einer Probe.

Endotoxin (ET) bezeichnet eine Familie von Lipopolysacchariden, die zusammen mit Proteinen und Phospholipiden die äußere Zellwand Gram-negativer Bakterien bilden. Endotoxine kommen ausschließlich in dieser Bakteriengruppe vor und spielen eine wichtige Rolle in der Organisation, Stabilität und Barrierefunktion der äußeren Membran. Zahlreiche Bakteriophagen nutzen Endotoxin bzw. allgemein Lipopolysaccharid zur spezifischen Erkennung ihrer Wirtsbäkterien.

Alle Endotoxinvarianten bestehen aus einem Heteropolysaccharid, das kovalent an Lipid A gebunden ist. Lipid A verankert Endotoxin in der äußeren Bakterienmembran. Das Heteropolysaccharid, das aus einem Herzoligosaccharid und dem O-Antigen besteht, zeigt in die umgebende Lösung und bestimmt die serologische Identität des Bakteriums. Das O-Antigen besteht aus repetitiven Oligosaccharideinheiten, deren Zusammensetzung stammspezifisch ist. Charakteristische Bausteine des Herzoligosaccharids sind 2-Keto-3-desoxyoctonsäure (KDO) und L-Glycero-D-manno-heptose (Hep).

Der konservativste Teil von Endotoxin verschiedener Gattungen ist das Lipid A. Ähnlich konserviert wie Lipid A ist die innere Herzregion, die äußere Herzregion weist bereits eine höhere Variation auf. Die innere Herzregion, KDO und Lipid A selbst tragen mehrere Phosphatgruppen als Substituenten und sind so für die negative Ladung von Endotoxin verantwortlich. Darüber hinaus können die Phosphatgruppen am Lipid A und der Herzregion variabel mit Arabinose, Ethanolamin und Phosphat substituiert sein. Einzelne Saccharidbausteine des O-Antigens sind acetyliert, sialysiert oder glycosyliert. Das O-Antigen variiert außerdem bezüglich der Anzahl repetitiver Einheiten, weshalb die Endotoxin-Population jedes Bakteriums eine gewisse Heterogenität aufweist.

Endotoxine sind Biomoleküle, die ohne entsprechende Vorsichtsmaßnahmen in praktisch allen wässrigen Lösungen vorzufinden sind. Endotoxine können bei Mensch und Tier zu Sepsis, einer starken Fehlreaktion des Immunsystems führen. Daher sind z.B. bei der Herstellung von Pharmaproteinen Verunreinigungen mit Endotoxin exakt nachzuweisen und in der Folge komplett zu entfernen. Endotoxin stellt ein Problem bei gentechnisch hergestellten Arzneimitteln, Gentherapeutika oder Substanzen dar, die in Mensch oder Tier (z.B. Tiermedizinische Behandlung oder bei Tierversuchen) injiziert werden. Doch nicht nur bei medizinischen, sondern auch bei Forschungsanwendungen, wie bei Transfektionsexperimenten von Säugerzellen kann eine Hemmung bzw. ein Senken der Transfektionseffizienz durch Endotoxin beobachtet werden.

Um Proteine im Rahmen von klinischen Studien einsetzen zu können, verlangen die europäische und die amerikanische Pharmacopeia, dass die Proteine bestimmte Grenzwerte an Endotoxinbelastung unterschreiten (z.B. Immunserum Globulin ≤0,91 EU/ml , dies entspricht ≤ 5 EU/kg Körpergewicht & Stunde (Dosis = EU/kg * h); EU = Endotoxin Unit; FDA (Food and Drug Administration): Guideline on Validation of LAL as End Product). Falls ein Medikament bzw. darin enthaltene Proteine eine zu hohe Endotoxinbelastung aufweisen, kann dies bis zum Tod des Probanden führen. Die fehlgeleitete Immunabwehr schädigt durch eine Überreaktion den Patienten. Dies kann zu Gewebeentzündungen, Blutdruckabfall, Herzrasen, Thrombose, Schock etc. führen. Bereits eine länger anhaltende Endotoxin-Exposition in Picogramm-Mengen kann zu chronischen Nebenwirkungen wie z.B. Immunschwächen, septischen Symptomen etc. führen. Im Rahmen der Substanzherstellung wird daher, insbesondere bei Prozessen unter "Good Manufacturing Practice" (GMP) Bedingungen, versucht, Endotoxin soweit wie möglich abzureichern. Allerdings ist die Endotoxin-Entfernung bei Proteinen, Polysacchariden und DNA problematisch. Gerade bei Proteinen gibt es große Probleme durch deren intrinsische Eigenschaften wie Ladungszustand oder Hydrophobizität, die eine Endotoxinentfernung nahezu verhindern bzw. zu großen Produktverlusten bei der Entfernungsprozedur führen können.

Derzeit sind vier Verfahren zum Endotoxin-Nachweis in biologischen Lösungen beschrieben, wobei nur die beiden ersten Verfahren von der FDA zugelassen sind. 1. "Rabbit Pyrogen Testing": Ein Verfahren, bei dem einem lebenden Kaninchen eine Endotoxin-Lösung injiziert und damit eine Immunreaktion ausgelöst wird. Diese Endotoxin-verursachte Immunantwort wird über die Entwicklung von Fieber nachgewiesen. 2. Deutlich besser standardisierbar ist der "Limulus Amoebocyte Lysate (LAL)" - Test, der derzeit am häufigsten verwendete Test (BioWhittaker, Inc., Charles River, Inc., Associates of Cape Cod, Inc., alle USA). Bei diesem Verfahren wird die Verklumpung des Blutes des Pfeilschwanzkrebses (Limulus polyphemus) nach Endotoxin-Kontakt gemessen. 3. Der InVitro Pyrogen Test basiert auf dem Nachweis von Interleukins-1β in menschlichem Blut, das an der Fieberinduktion beteiligt ist. Der Test besteht aus einem Inkubationsschritt von menschlichem Blut mit der zu untersuchenden Lösung und der anschließenden Detektion des Interleukins über Antikörper. 4. Eine weitere Möglichkeit ist der Einsatz eines speziellen Zellkultursystems (Sterogene Inc., USA), mit dem die Aktivierung von Monozyten über die Entstehung bestimmter Zytokine verfolgt wird.

Die beiden erstgenannten Verfahren sind jedoch sehr teuer und durch den großen Bedarf an Versuchstieren bzw. an Blut des sehr seltenen Pfeilschwanzkrebses nicht zuletzt aus Tierschutzgründen bedenklich. Der LAL-Test kann zwar auch miniaturisiert und automatisiert werden, hat aber aufgrund geringer Stabilität der Komponenten massive Nachteile in der Anwendung. Eine einmal geöffnete LAL-Lösung muß direkt weiterverarbeitet und aufgebraucht werden, da die Komponenten innerhalb weniger Stunden aggregieren. Der InVitro Pyrogen Test benötigt möglichst frisches menschliches Blut und ist relativ zeitaufwändig, da die Produktion des Interleukins etwa 10 bis 24 Stunden benötigt. Neben Endotoxinen können mit dem Pyrogen Test auch andere Pyrogene erkannt werden. Dieser Test wird jedoch in erster Linie als Ersatz für den "Rabbit Pyrogen Test" verwendet. Für alle Testverfahren ist geschultes Personal nötig und die Verfahren sind sehr störanfällig, weil z.B. das Immunsystem von Kaninchen auf die gleiche Endotoxindosis durchaus unterschiedlich reagieren kann. Das Zellkultur-Verfahren der Firma Sterogene ist, wie alle Zellkulturverfahren, ebenfalls sehr aufwändig und weist Probleme bei der Standardisierung auf.

Insgesamt kann festgestellt werden, dass es kein einfach handhabbares und kostengünstiges Verfahren zum Endotoxinnachweis gibt und die derzeit eingesetzten Methoden eine Reihe von Nachteilen aufweisen. Es besteht daher der Bedarf für ein Verfahren, das diese Nachteile umgeht.

Daher liegt der Erfindung die Aufgabe zu Grunde, ein Verfahren bereitzustellen, das Endotoxine schneller, einfacher und standardisierter in Lösungen und Proben nachweisen kann.

Die Aufgaben werden durch den in den Patentansprüchen definierten Gegenstand gelöst.

Die nachfolgenden Figuren erläutern die Erfindung.

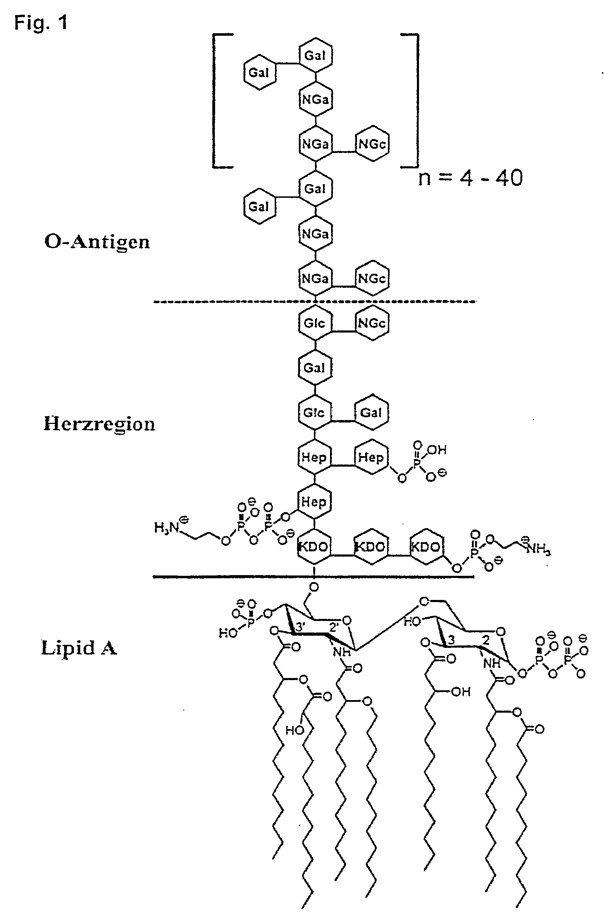

Fig. 1 zeigt eine schematische Übersicht der chemischen Struktur von Endotoxin aus E. coli 0111:B4. Hep = L-Glycero-D-manno-heptose; Gal = Galactose; Glc = Glucose; KDO = 2-Keto-3-desoxyoctonsäure; NGa = N-Acetyl-galactosamin; NGc = N-Acetylglucosamin.

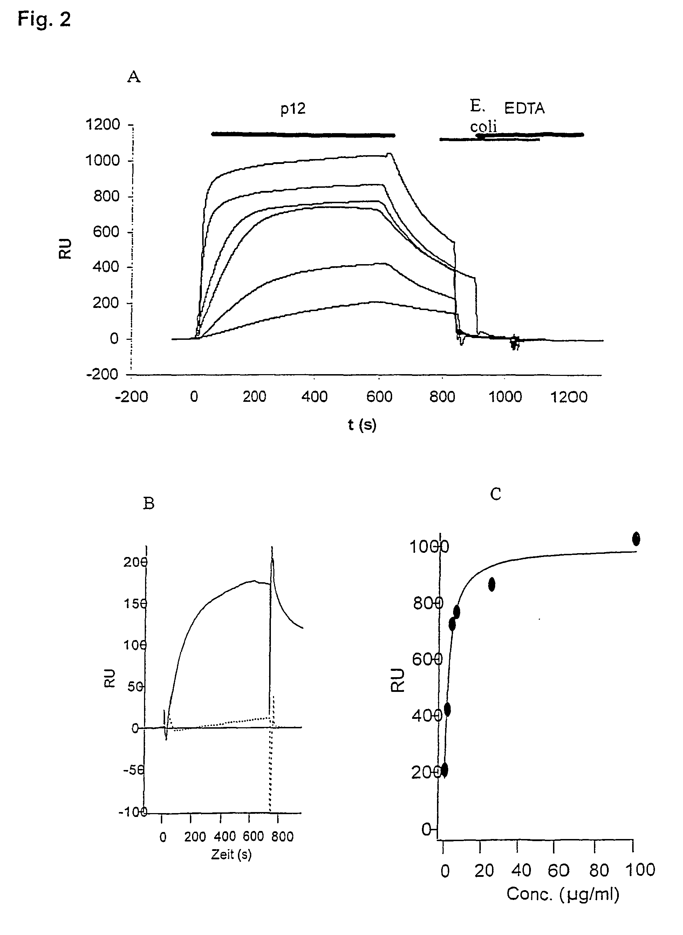

Figur 2 zeigt Ergebnisse von Oberflächen-Plasmon-Resonanz Messungen. (A) Resonanzkurven, die als Antwort auf Injektion von verschiedenen (je in µg/ml: 100; 25; 6,25; 4; 1,56; 0,4) p12-Konzentrationen (_) gemessen wurden. Die Bindung erfolgt an Endotoxin von E. coli D21f1, das auf einem hydrophoben HPA-Chip immobilisiert wurde. Die Injektion von p12 und EDTA (5 mM) wird durch Balken über den Kurven markiert. Puffer: 20 mM Tris, 150 mM NaCl, pH 8.0. (B) Gleichgewichtsresonanzwerte für die Bindung von p12 an immobilisiertes Endotoxin wurden etwa 600 s nach Beginn der p12 Injektion gemessen und gegen die dazugehörigen p12-Konzentration aufgetragen. Die durchgezogene Linie zeigt einen Fit der Langmuirschen Adsorptionsisotherme (RU = RUₘₐₓ*[p12]/([p12]+K_{d})) an die Daten. (C) Bindung von E. coli an biotinyliertes p12, das auf Streptavidin-Chips immobilisiert wurde. E. coli D21eS (_), dessen innerere Herz-Region vollständig ist, an p12. Dagegen bindet E. coli D21f2 (----), der eine stark verkürzte Herz-Region besitzt, nicht an p12. Die Messungen wurden in PBS durchgeführt.

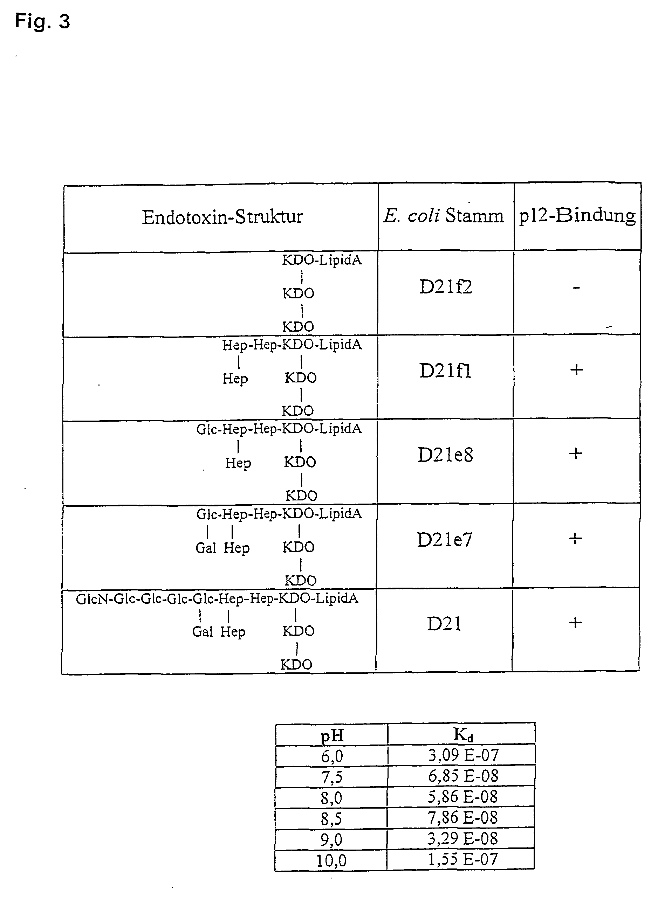

Figur 3 zeigt schematisch die Struktur der Endotoxin-Herzregion verschiedener E.coli-Mutanten.

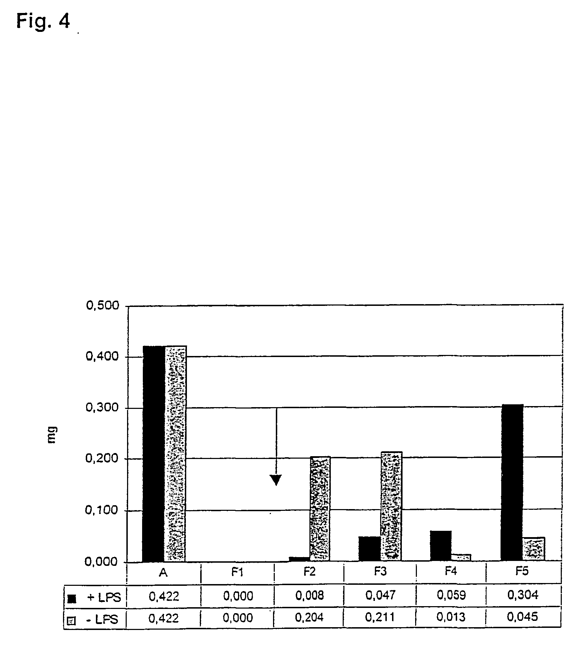

Figur 4 zeigt in einem Balkendiagramm das Bindungsverhalten des Bakteriophagenschwanzproteins p12 an Endotoxin, das mittels Polymyxin B an Chromatographiesäulen (0.5 ml) gebunden worden war. Es wurden jeweils 2 Polymyxin B Säulen mit Endotoxin von E. coli 055:B5 (10⁶ EU/ml) gespült (+LPS, schwarze Balken) und 2 Säulen mit Wasser gewaschen (- LPS, gestreifte Balken). Die Menge des Bakteriophagenschwanzproteins p12 wurde gegen die Fraktionen des Chromatographielaufs aufgetragen. Jeder Balken zeigt die Mittelwerte, die aus zwei parallelen Chromatographieläufen ermittelt wurden. Der erste Balkenpaar (A) zeigt die aufgetragene p12 Menge und das zweite die Fraktion 1 (F1), eine Kontrollfraktion vor dem Auftrag von p12 auf die Säule. Der Pfeil markiert den Auftrag von p12 auf die Säule. Die Fraktionen 2 - 5 wurden nach dem Auftrag gesammelt. Die Konzentration von p12 wurde durch Absorptionsmessung bei 280 nm ermittelt. Das Fraktionsvolumen für die Fraktionen 1-4 betrug 1 ml und 2 ml für Fraktion 5. Die Regeneration der Säule in Fraktion 5 erfolgte durch Zugabe von 2 mM EDTA zum Laufpuffer (20 mM Hepes, 150 mM NaCl, 0,1 mM CaCl₂, pH 7,5). Das Bakteriophagenschwanzprotein p12 wurde auf den Säulen, die vorher mit Endotoxin beladen worden waren, zurückgehalten, während es durch die Säulen, die kein Endotoxin enthielten, ohne Verzögerung durchlief.

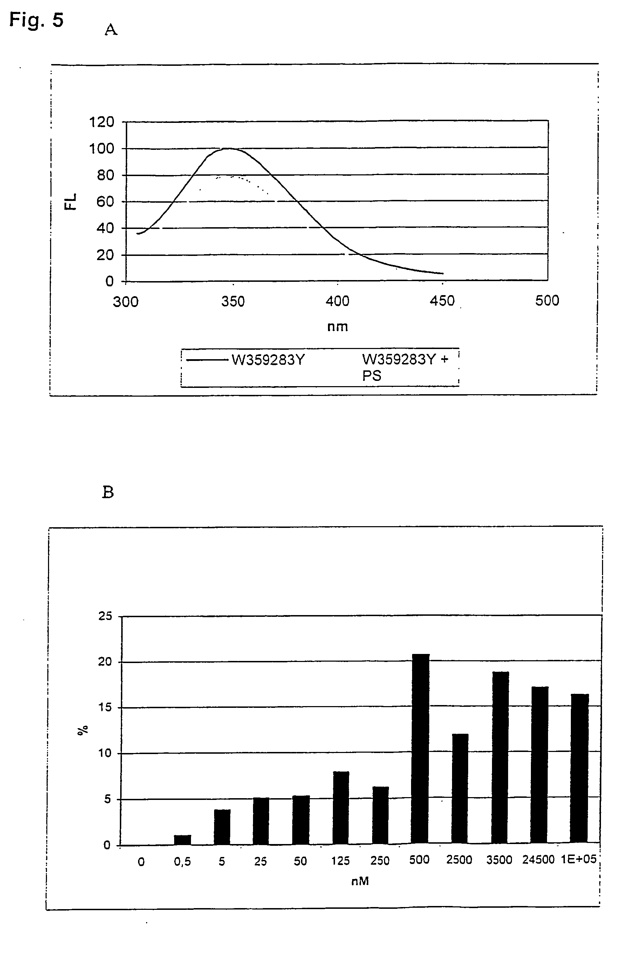

Figur 5A und B zeigt in einer Graphik die Abnahme der Fluoreszenz der T4p12-Mutante W359_283Y nach Zugabe von Endotoxin-Polysaccharid (aus Salmonella thyphimurium). A) Die Fluoreszenz der p12-Mutante W359_283Y (40 µg/ml) im Bereich von 305-450 nm wurde nach Anregung bei 295 nm gemessen. Nach Zugabe von 3 µl Polysaccharid (10 mg/ml) (graue Kurve). zu 120 µl Lösung mit p12-Mutante W359_283Y konnte im Vergleich zur unbehandelten Probe (schwarze Kurve) eine Abnahme der Fluoreszenz beobachtet werden Die Kurven wurden gegen Kontrollmessungen ohne die p12 Mutante korrigiert. Figur B) zeigt die prozentuale Abnahme der Fluoreszenz der p12 Mutante W359_283Y in Abhängigkeit von der Konzentration des applizierten Endotoxin-Polysaccharids. Die Anregungswellenlänge war 295 nm und die Emissionswellenlänge 350 nm. Es wurde die p12 Mutante W359_283Y (200 µg/ml oder 3.6 µM) vorgelegt und mit Endotoxin-Polysaccharid titriert. Auf der X-Achse sind die Endkonzentrationen des Endotoxin-Polysaccharids aufgetragen. Die Messwerte wurden gegen Kontrollmessungen ohne die p12 Mutante W359_283Y korrigiert. Ab einer Polysaccharidkonzentration von 500 nM konnte eine deutliche Abnahme der Fluoreszenz gemessen werden.

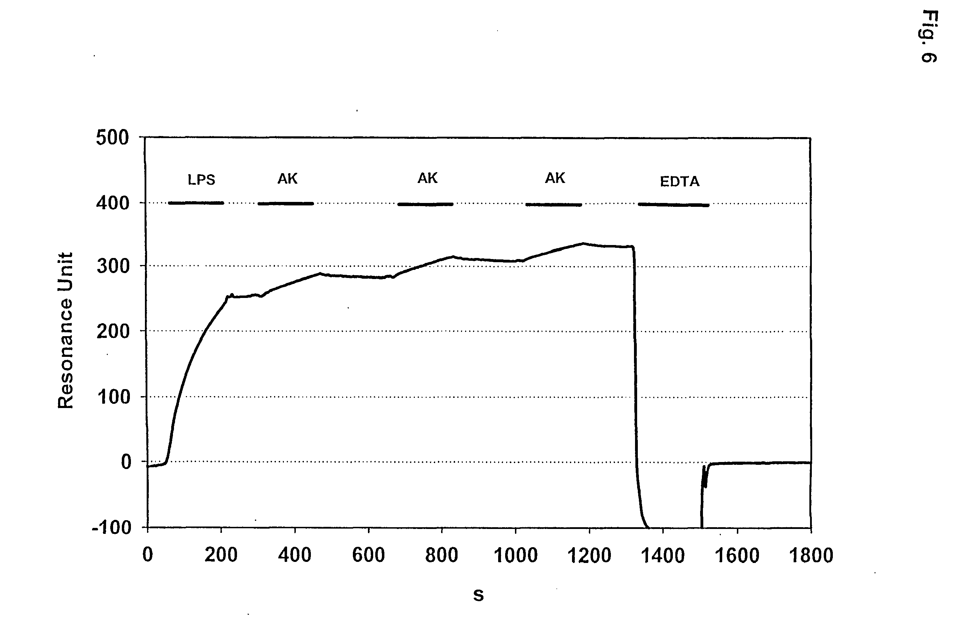

Figur 6 zeigt die Bindung eines anti-Lipid A Antikörpers an Lipopolysaccharid das über T4p12 auf einer Oberfläche gebunden ist. Die Bindung wurde durch Messung des Oberflächen-Plasmon-Resonanz Signals mit einem Biacore J beobachtet. Zunächst wurde T4p12 auf einer Durchflusszelle (CM-5 Chip von Biacore) über primäre Aminogruppen entsprechend den Vorschriften des Herstellers kovalent immobilisiert. Anschließend wurde Lipopolysaccharid von *E. coli* O55:B5 (LPS, 0.1 mg/ml) injiziert. Die Injektionsphasen sind durch Balken über der Messkurve markiert. Die Zunahme des Resonanzsignals zeigt die Bindung von Lipopolysaccharid an T4p12. Auch nach Beendigung der Injektion bleibt das Resonanzsignal erhöht und damit das Lipopolysaccharid an die Oberfläche gebunden. Anschließend wurde drei mal ein Antikörper (AK) gegen Lipopolysaccharide injiziert (2 µg/ml, polyklonaler Antikörper gegen Lipid A von Accurate Chemical & Scientific Corporation). Die Zunahme des Resonanzsignals bei jeder Injektion zeigt die Bindung des Antikörpers. Durch Zugabe von EDTA (EDTA) konnte die Bindung von Lipopolysaccharid an T4p12 gelöst werden. Das Resonanzsignal kehrt auf das Ausgangsniveau zurück. Eine zweite Durchflusszelle blieb unbehandelt und diente als Referenz. Die dargestellte Kurve zeigt die Differenz des Signals von der Messzelle und der Referenzzelle.

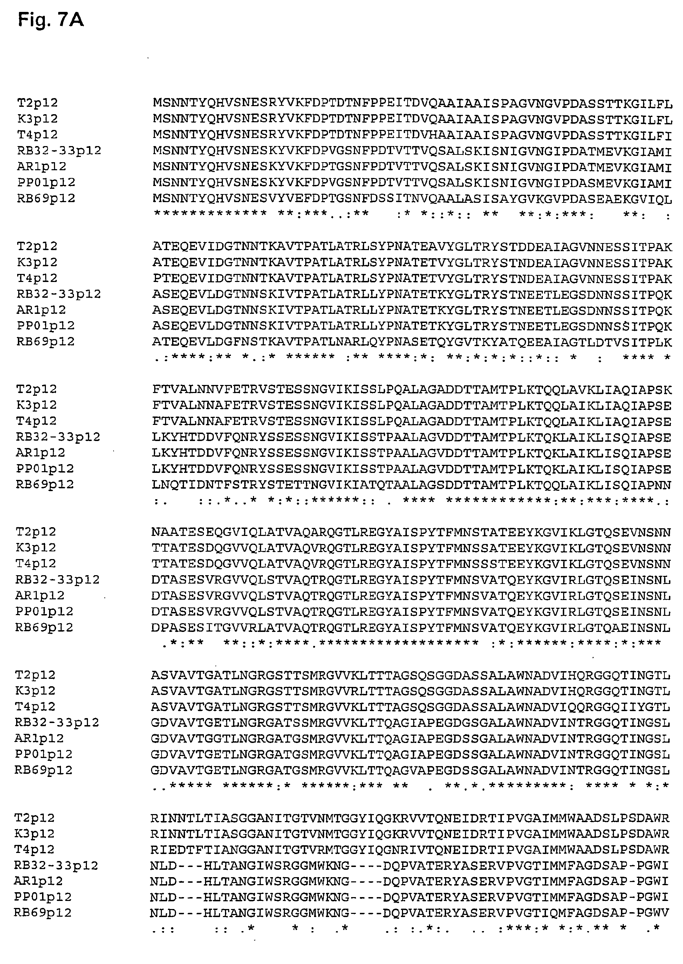

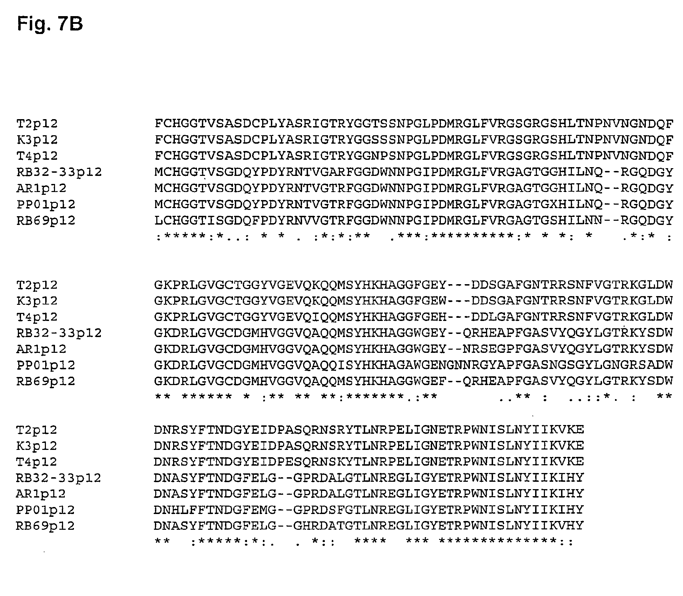

Figur 7 zeigt einen Vergleich verschiedener p12 Bakteriophagenschwanzproteine auf Aminosäureebene. Die Proteine stammen von Phagen der Myrovidiaefamilie und besitzen eine Homologie von mindestens 60% zu T4p12. Die für den Vergleich verwendeten p12 Proteine stammen von den Phagen T2 (NCBI-Datenbank Accession Nr: CAA39905; SEQ ID NO:9), T4 (AAD42417; SEQ ID NO:10), PP01 (BAD20635; SEQ ID NO:11), RB69 (AAP76072; SEQ ID NO:12) und AR1 (AAN03609; SEQ ID NO:13). Proteine der Phagen K3 (Btuda M.R. et al., Biol. Chem. (2000) 381, 225-258), RB32-33 und Ox2 weisen eine ähnliche Homologie zu den oben genannten Phagen auf.

Der Begriff "Probenmaterial" oder "Probe" wie hier verwendet umfasst sämtliche Lösungen, in denen Endotoxine nachgewiesen werden sollen werden sollen. Beispielhaft für Lösungen ist die folgende Aufzählung: wässrige Lösungen und Gemische aus Wasser und organischen Lösungsmitteln, Blut, Blutprodukte, Plasma, Serum, Urin, Medien. Beispielhafte Lösungen sind ferner solche, in denen feste zu untersuchende oder zu reinigende niedermolekulare und/oder hochmolekulare Substanzen gelöst wurden, beispielsweise, Zucker, Salze, Antibiotika, Proteine, DNA, RNA, Lebensmittel, Arzneimittel, Impfstoffe, oder organische und anorganische Chemikalien wie z.B. NaCl, MgCl₂, Purine oder Pyrimidine.

Der Begriff "Endotoxin" wie hier verwendet bezeichnet das bakterielle Lipopolysaccharid, das Bestandteil der äußeren Membran gram-negativer Bakterien ist. Endotoxin bezeichnet nicht nur das vollständige Lipopolysaccharid sondern auch die einzelnen Bestandteile z.B. das Lipid A, die innere oder äußere Herzregion.

Der Begriff "Bakteriophagenschwanzprotein" wie hier verwendet bezeichnet solche Proteine, die in Bakteriophagen vorkommen und Endotoxine binden können. Üblicherweise sind diese Proteine im Bakteriophagenschwanz lokalisiert, können jedoch auch auf dem Bakteriophagenkopf oder bei Bakteriophagen ohne Schwanz auf der normalen Bakteriophagenhülle lokalisiert sein. Der Begriff Bakteriophagenschwanzprotein umfasst sowohl kurze als auch lange Bakteriophagenschwanzproteine. So können Bakteriophagen mit einer Basisplatte (z.B. Myoviridae wie T4-ähnliche Phagen) unterschiedliche Bakteriophagenschwanzproteine, so genannte lange oder kurze Bakteriophagenschwanzproteine aufweisen, die auch unterschiedliche Spezifität für Strukturen von Bakterienmembranen besitzen.

Der hier verwendete Begriff "Homologie" bezeichnet eine signifikante Ähnlichkeit einer Aminosäuresequenz zu einer Vergleichssequenz oder Teilen davon. Die Homologie einer Sequenz wird unter Zuhilfenahme des Similaritätsalgorithmus BLAST (Basic Local Alignment Search Tool, Altschul et al., Journal of Molecular Biology 215, 403-410 (1990*)* bestimmt. Als signifikant ähnlich werden, wie hier verwendet, Sequenzen bezeichnet, die eine Homologie von mindestens 60% aufweisen oder die z.B. unter Verwendung von Standardparametern im BLAST-Service des NCBI ein Signifikanzniveau (E-Value oder Probability) von P < 10⁻⁵ aufweisen, wenn Sie mit den Vergleichssequenzen verglichen werden.

Der Begriff "unspezifische Immobilisierung" oder "ungerichtete Immobilisierung" wie hier verwendet bedeutet, dass die Kopplung eines Proteins an eine Oberfläche über Proteinreste (z.B. primäre Amine) erfolgt, die über die gesamte Proteinoberfläche verteilt sein können. Die Auswahl der für die Kopplung des einzelnen Proteinmoleküls verwendeten Gruppe ist zufällig.

Der Begriff "Oberfläche" oder "Träger" wie hier verwendet umfasst alle Materialien, an die eine Kopplung oder Adhäsion eines Proteinmoleküls möglich ist, wie z.B. Glasoberflächen, Chromatographiematerialien, z.B. Agarose oder Sepharose, Plastikoberflächen, z.B. Polystyrol oder Polypropylen, Filtermaterialien, z.B. Cellulose.

Der Begriff "gerichtete Immobilisierung" wie hier verwendet bedeutet, dass die Kopplung über Aminosäurereste oder andere Reste (z.B. Glykosyslierungen des Proteins) erfolgt, deren Position im Protein (z. B. N- oder C-terminal) bekannt ist. Die Auswahl dieser Gruppen für die Kopplung erfolgt durch die Auswahl geeigneter Reaktionspartner/Linker, die bevorzugt mit diesen Resten reagieren (z.B. Kopplung von Sulfhydrylresten an Iodoacetatreste; Iodoacetat reagiert tausendmal schneller mit Sulfhydrylresten als mit Aminoresten).

Ein Aspekt betrifft ein Verfahren zum Nachweis von Endotoxin, umfassend die Schritte:
a) Inkubieren einer Probe mit Bakteriophagenschwanzproteinen, und anschließend
b) Nachweis von an Bakteriophagenschwanzproteine gebundenem Endotoxin mittels spektroskopischer Verfahren, ELISA, chemischer oder enzymatischer Nachweisreaktion von Endotoxine oder abgespaltenen Endotoxinkomponenten, oder mittels Kapazitätsmessung.

Gegebenenfalls wird nach Schritt a) und vor Schritt b) ein zusätzlicher Schritt a') Abtrennung von Bakteriophagenschwanzprotein-Endotoxin-Komplex von der Probe eingeführt.

Der Nachweis mittels spektroskopischer Verfahren kann z.B. mit Fluoreszenzemission, Fluoreszenzpolarisation, Absorption oder Circulardichroismus, der Nachweis mittels Kapazitätsmessung kann z.B. mittels elektrischer Signale durchgeführt werden. Die aufgeführten Nachweise können ferner mit einem Kompetitionsnachweis kombiniert werden.

Vorzugsweise beschrieben ist ein Verfahren, bei dem nach Abtrennung des in Schritt a) gebildeten Bakteriophagenschwanzprotein-Endotoxin-Komplexes von der Probe, der Nachweis der Endotoxine über immunologische, chemische oder enzymatische Reaktionen erfolgt. Dazu können die Bakteriophagenschwanzproteine mit Hilfe spezieller Liganden, wie z.B. Biotin, Strep-Tag oder His-Tag, an die entsprechenden Träger z.B. Sepharosen oder magnetische Beads, die mit Streptavidin oder Streptactin beschichtet sind, gebunden werden. Anschließend kann, falls gewünscht, eine Abtrennung der grobkörnigen Träger durch Filtration, Zentrifugation oder magnetische Separation von der Probe erfolgen. Gewünscht ist die Abtrennung insbesondere, wenn die der Bakteriophagenschwanzprotein-Endotoxin-Komplex auf einer Oberfläche fixiert ist, die in den verwendeten Nachweisen nicht eingesetzt werden kann.

Der immunologische Nachweis erfolgt z.B. über die Bindung von Endotoxin spezifischen Antikörpern an die Endotoxine, Bindung eines Sekundärantikörpers an den ersten Antikörper und anschließender Detektion über eine enzymtische Reaktion, die durch ein an den Sekundärantikörper fusioniertes Enzym katalysiert wird (ELISA).

Der Endotoxin-Nachweis kann auch nach chemischer Spaltung des Endotoxins mittels Säure oder Base und anschließender Detektion einzelner Endotoxinbestandteile, wie der 2-Keto-Deoxyoctonsäure, den Heptosen (Lee C.-H., Tsai C.-M., Analytical Biochemistry, 1999; 267: 161-168) oder den Hydroxy-Fettsäuren (Lyngby J., Olsen L.H., Eidem T. Lundanes E. Jantzen E., Biologics, 2002; 30: 7-13) erfolgen.

Die vorliegende Erfindung betrifft ein Verfahren zum Nachweis von Endotoxin, umfassend die Schritte:
a) In Kontakt bringen einer Probe enthaltend Endotoxine mit einem Träger, anschließend
b) Inkubieren von Bakteriophagenschwanzproteinen an die auf dem Träger immobilisierten Endotoxin, und
c) Nachweis der Bakteriophagenschwanzproteine mittels spektroskopischer Verfahren, ELISA, chemischer oder enzymatischer Nachweisreaktion von Endotoxinen oder abgespaltenen Endotoxinkomponenten, oder mittels Kapazitätsmessung.

Gegebenenfalls wird nach Schritt b) ein weiterer Schritt b') Abtrennen der gebundenen Bakteriophagenschwanzproteine vom Endotoxin, durchgeführt.

Insbesondere bevorzugt sind die p12 Proteine der Phagen K3, T2, T4, Ox2, RB32-33, AR1, PP01 oder RB69.

Bevorzugt ist ein Verfahren, bei dem nach der Bindung von Endotoxin an eine Oberfläche, die endotoxinbindende Liganden, wie Polymyxin B, Poly-L-Lysin, Chitosan und ähnliche tragen, Bakteriophagenschwanzproteine an die immobolisierten Endotoxine binden und diese Bakteriophagenschwanzproteine über eine anschließende Enzymreaktion nachgewiesen werden. Die Bakteriophagenschwanzproteine können mittels ELISA nachgewiesen werden, der spezifisch für das Bakteriophagenschwanzprotein ist, oder über Enzyme, die mit dem Bakteriophagenschwanzprotein gentechnisch fusioniert oder über chemische Reaktionen gebunden wurden. Bei den Enzymen handelt es sich z.B. um Alkalische Phosphatase, Peroxidase, oder andere.

Vorzugsweise wird vor dem Inkubationsschritt der erfindungsgemäßen Verfahren die Ionenzusammensetzung der zweiwertigen Ionen z.B. Ca²⁺, Mg²⁺ und/oder der pH-Wert eingestellt, um eine optimale Endotoxin-Bakteriophagenschwanzprotein-Bindung zu erhalten. Ferner bevorzugt wird bei oder nach der Inkubation eine "Demaskierung" des gebundenen Endotoxins durch Zugabe von Detergentien und/oder Salzen, z.B. Tween, Triton, NaCl oder Ammoniumsulfat, oder anderer Substanzen, z.B. Chitosan, Zucker oder Lipide, die ein Ablösen der Endotoxine von z.B. Proteinen oder Nukleinsäuren beschleunigen.

Das für den Nachweis von Endotoxin verwendete Bakteriophagenschwanzprotein kann ein natürlicherweise vorkommendes oder ein molekularbiologisch oder biochemisch modifiziertes sein. Insbesondere bevorzugt sind Bakteriophagenproteine, die an sehr konservierte Bereiche von Endotoxin, nämlich an die Herzregion des LPS oder an Lipid A binden. Insbesondere bevorzugt sind die kurzen Bakteriophagenschwanzproteine vorzugsweise von Myoviridae-Phagen. Es können jedoch auch die Endotoxin-bindenden Proteine eines Bakteriophagenkopfes oder der normalen Bakteriophagenhülle von Bakteriophagen ohne Schwanz verwendet werden. Ganz besonders bevorzugt sind Bakteriophagenschwanzproteine mit einer Homologie von mindestens 60% auf Aminosäureebene zu dem p12 Protein von T4. Das Bakteriophagenschwanzprotein kann aus verschiedenen Gründen gentechnisch und/oder biochemisch modifiziert sein. Für die erfindungsgemäßen Verfahren können jedoch nicht nur die natürlicherweise vorkommenden Bakteriophagenschwanzproteine verwendet werden, sondern auch deren Varianten. Varianten bedeutet im Sinne der vorliegenden Erfindung, dass die Bakteriophagenschwanzproteine eine veränderte Aminosäuresequenz aufweisen. Diese können durch Screening der natürlich auftretenden Varianten, oder durch Zufalls-Mutagenese oder gezielte Mutagenese, aber auch durch chemische Modifikation erhalten werden. Die für die erfindungsgemäßen Verfahren verwendeten Bakteriophagenschwanzproteine können durch eine gezielte oder zufällige Mutagenese in ihrer Spezifität bzw. ihren Bindungseigenschaften an Trägerstrukturen angepaßt werden. Diese Bindung an die Träger kann fest, z.B. kovalent oder über eine spezifische oder unspezifische Biotinylierung erfolgen, aber auch reversibel z.B. über eine reduzierbare Disulfidbrücke. Ferner kann durch eine Modifikation die Stabilität erhöht werden. Durch die molekularbiologische oder chemische Mutagenese werden Mutationen eingeführt, die Aminosäureadditionen, -deletionen, -substitutionen oder chemische Modifikationen sein können. Diese Mutationen können eine Veränderung der Aminosäuresequenz in der Bindungsregion der Bakteriophagenschwanzproteine bewirken, mit dem Ziel, Spezifität und Bindungsaffinität an Testbedürfnisse anzupassen, z.B. die Bindung der Endotoxine an die Bakteriophagenschwanzproteine zu erhöhen oder irreversibel zu machen, um den Nachweis zu verbessern. Darüber hinaus kann eine gentechnische oder biochemische Modifikation der Phagenproteine durchgeführt werden, mit dem Ziel, die gegebenenfalls vorhandene enzymatische Aktivität auszuschalten, um dadurch die Bindung zu verbessern oder irreversibel zu machen. Weiterhin kann eine gentechnische oder chemische Modifikation der Phagenproteine durchgeführt werden, um die vorhandenen physikalischen Eigenschaften des Proteins wie Löslichkeit Thermostabilität usw. im Sinne des erfindungsgemäßen Verfahrens anzupassen.

Außerdem kann eine Verknüpfung der Bakteriophagenschwanzproteine mit enzymatisch wirksamen Proteinen erfolgen, um die Bakterienschwanzproteine sensitiver nachweisen zu können. Enzymatisch wirksame Proteine wie Alkalische Phosphatase oder Meerrettich Peroxidase, für die es kommerzielle Substrate gibt, können mittels chemischer Kopplungsmethoden oder über gentechnologische Fusion mit den Bakteriophagenschwanzproteinen verknüpft werden. Die enzymatische Reaktion, die über diese Proteine eingeführt wird, erhöht die Sensitivität des Nachweises deutlich.

Arbeiten zur Aufklärung der dreidimensionalen Struktur von T4 p12 haben gezeigt, dass bei erhöhter Temperatur proteolytische Fragmente von 33 kDa und 45 kDa erzeugt werden können, die N- und C-terminal (33 kDa) bzw. nur N-terminal (45 kDa) verkürzt sind. Im Gegensatz zu dem 33kDa Fragment ist das 45kDa Fragment noch in der Lage an Bakterien zu binden (Thomassen, E., et al., Mol. Biol.; 331: 361-373, 2003). Demzufolge ist der C-Terminus an der Zellbindung beteiligt. Daher kann durch eine N-terminale Modifikation eine gerichtete Bindung an Oberflächen durchgeführt werden und somit letztendlich die Bindung von Endotoxin indirekt optimiert werden. Darüberhinaus ist eine direkte Optimierung der Endotoxin-Bindung möglich.

Die Modifikation kann ferner insbesondere den Zweck haben, einen direkten Nachweis z.B. mittels Messung der Tryptophanfluoreszenz zu ermöglichen. Beispielsweise besitzt T4 p12 fünf Tryptophan-Reste. Das Fluoreszenzspektrum des nativen Proteins deutet darauf hin, dass diese Reste weitestgehend lösungsmittel-unzugänglich sind. Aus einer Vielzahl von wissenschaftlichen Arbeiten ist bekannt, dass fast immer aromatische Aminosäuren an der Bindung von Zuckerresten, wie sie auch in Endotoxin vorkommen, beteiligt sind. Die Bindung der Zuckerreste an Proteine kann durch einen Quench der Trypthophanfluoreszenz, bzw. gegebenenfalls auch zusätzlich durch eine Veränderung des Fluoreszenzmaximums verfolgt werden. Eigene Arbeiten lassen vermuten, dass die ungünstige Verteilung der Fluorophore des natürlichen p12 eine Ausnutzung der Fluoreszenz-Eigenschaften von p12 zur Bindungsmessung verhindert. Die Fluoreszeneigenschaften von p12 werden durch die fünf Tryptophanreste dominiert, deren Fluoreszenz durch die Zugabe von Endotoxin nicht messbar verändert wird. Diese Daten lassen erwarten, dass eher Tyrosinreste als Tryptophanreste an der Bindung beteiligt sind, deren Signaländerung vor dem hohen Tryptophan-Hintergrund nicht sichtbar gemacht werden kann. Auf der Basis der Proteolyseergebnisse kommen sechs Tyrosine am C-Terminus von p12 für den Endotoxin-Nachweiskit in Frage, die entsprechend "sichtbar" gemacht werden können. Durch einen selektiven molekularbiologischen Austausch der fünf Tryptophan-Reste gegen Tyrosine werden in einem ersten Schritt die spektroskopischen Eigenschaften so gezielt verändert, dass die Endotoxin-Bindung per Fluoreszenzsignaländerung eines einzelnen Tryptophanrestes messbar ist. Anschließend wird durch einen gezielten Austausch von jeweils einem der sechs Tyrosine im C-terminalen Bereich gegen einen Tryptophanrest die Intensität des messbaren Signals signifikant erhöht, um für die Entwicklung eines Endotoxin-Nachweiskits attraktive Signalunterschiede zu erhalten. Wie für das p12 Protein von T4 gezeigt, können auch andere kurze Bakteriophagenschwanzproteine wie z.B. solche aus Myoviridae Phagen wie T4, T2, K3, Ox2, RB32-33 oder RB69 entsprechend modifiziert werden. Auch hier können jedoch ebenfalls die Endotoxin-bindenden Proteine eines Bakteriophagenkopfes oder der normalen Bakteriophagenhülle von Bakteriophagen ohne Schwanz verwendet werden, insbesondere von PhiX 174.

Welche Bakteriophagenschwanzproteine verwendet werden, hängt davon ab, welche Endotoxine nachgewiesen werden sollen. Bereits jetzt steht eine große Zahl bekannter Bakteriophagen für einen Großteil der bisher beschriebenen Bakterien zur Verfügung und kann für die erfindungsgemäßen Verfahren verwendet werden. Die Phagen und die entsprechenden Wirtsbakterien sind u.a. bei folgenden Stammsammlungen erhältlich: ATCC (USA), DSMZ (Deutschland), UKNCC (Großbritannien), NCCB (Niederlande) und MAFF (Japan); oder können durch mikrobiologische Standardmethoden aus Umweltproben isoliert werden. Bakteriophagenproteine können aus der Familie der Myoviridae kommen, also kurze Schwanzproteine sein, insbesondere aus der Gruppe der Pseudo-T-even, Schizo-T-Even oder der T-Even Phagen. Vorzugsweise werden für den erfindungsgemäßen Nachweis die kurzen Bakteriophagenschwanzproteine der Phagen K3, T2, T4, Ox2, RB32-33, AR1, PP01 und RB69 oder die Endotoxin-bindenden Proteine der Bakteriophagen ohne Schwanz z.B. von PhiX 174 verwendet.

Vorzugsweise stammen die Bakteriophagenschwanzproteine für die erfindungsgemäßen Verfahren von Bakteriophagen, deren Wirtsbakterien medizinisch oder biotechnologisch relevante Bedeutung haben, wie z.B. E. coli, das bei der Produktion rekombinanter Proteine oder von Nukleinsäuren für die Gentheraphie verwendet wird. Besonders bevorzugt sind Bakteriophagenschwanzproteine, die stark konservierte Bereiche von Endotoxin binden, wie z.B. die Herzregion oder Lipid A. Insbesondere bevorzugt sind T4p12 und T4p12-ähnliche Bakteriophagenschwanzproteine, z.B. T2-p12, K3-p12 (Burda-MR, Hindennach-I, Miller-S, Biol. Chem. 2000; 381: 255-258). Bei einer Kombination von Endotoxin-Verunreinigungen aus verschiedenen Wirtsbakterien kann für die erfindungsgemäßen Nachweise oder Abreicherungen eine Kombination der entsprechenden Endotoxin-erkennenden Bakteriophagenschwanzproteine eingesetzt werden.

Der Nachweis von Endotoxin in oder aus einer Probe erfolgt über die Bindung von Endotoxin an die Bakteriophagenschwanzproteine. Diese Bindung kann z.B. durch direkte Messung mittels spektroskopischer Verfahren, z.B. über Fluoreszenzemission, Fluoreszenzpolarisation, Absorption oder Circulardichroismus nachgewiesen werden. Darüber hinaus kann die Bindung durch elektrische Signale, z.B. eine Kapazitätsmessung sichtbar gemacht werden. Weiterhin kann die Bindung von Endotoxin an die Bakteriophagenschwanzproteine auch indirekt über Verdrängungsexperimente nachgewiesen werden.

Außerdem kann die Bindung von Bakteriophagenschwanzproteinen an Endotoxin nachgewiesen werden, indem die Endotoxine zunächst über andere endotoxinbindende Substanzen oder auch über ein zweites Bakteriophagenschwanzprotein auf einer Oberfläche immoblisiert werden, und anschließend ein anderes Bakteriophagenschwanzprotein an Endotoxin bindet. Nach dem Auswaschen von überschüssigem Bakteriophagenschwanzprotein wird anschließend die Menge des gebundenen anderen Bakteriophagenschwanzproteins quantifiziert. Dies erfolgt entweder über Antikörper gegen das andere Bakteriophagenschwanzprotein (ein sog. ELISA), oder über eine enzymatische Reaktion, die durch ein Protein katalysiert wird, das an das andere Bakteriophagenschwanzproteine fusionierte ist. Die Oberfläche kann dazu vorher mit Endotoxin bindenden Substanzen, wie z.B. Polymyxin B, Histidin, Histamin, Poly-L-Lysin, DEAE, Polyethylenimin, Deoxycholsäure, Poly γ-aminomethyl-L-glutamin, Poly Vinylalkohol, Poly-N,N-dimethylaminopropylacrlyamid, Dextran, Chitosan, und ähnliche beschichtet werden. Außerdem kann auch ein Bakteriophagenschwanzprotein für die Immobilisierung von Endotoxin verwendet werden. Der Nachweis von Endotoxin erfolgt dann mit einem zweiten Bakteriophagenschwanzprotein, das andere Endotoxin Bindungseigenschaften besitzt als das für die Immobilisierung benutzte Bakteriophagenschwanzprotein. Die Immobilisierung der Endotoxin bindenden Substanzen erfolgt dabei entweder durch Adhäsion, kovalente Kopplung, oder Bindung über spezielle Immobilisierungsgruppen, wie Biotin, Streptavidin, Strep-Tag, His-Tag, und vergleichbare Gruppen. Der Nachweis des Bakteriophagenschwanzproteins kann auch nach Ablösen des Proteins von der Oberfläche erfolgen.

Für die erfindungsgemäßen Nachweise können die Bakteriophagenschwanzproteine bei Bedarf einer Abtrennung der Bakteriophagenschwanzprotein-Endotoxin-Komplexe von der Probe auf geeigneten Oberflächen, z.B. Magnetpartikeln, Sepharosepartikeln, Agarosepartikeln, Mikrotiterplatten, Filtermaterialien oder Durchflußzellkammem, gekoppelt werden (indirekter Nachweis). Die Trägerstrukturen können z.B. aus Polystyrol, Polypropylen, Polycarbonat, PMMA, Celluloseacetat, Nitrozellulose, Glas, Silizium oder Agarose bestehen. Die Kopplung kann z.B. durch Adsorption oder kovalente Bindung erreicht werden.

Wichtig hierbei ist eine funktionelle Kopplung, d.h. Bakteriophagenschwanzproteine verfügen trotz Bindung an das Trägermaterial über für Endotoxin zugängliche Strukturen bzw. Bindestellen. Die Kopplung der Bakteriophagenschwanzproteine kann unspezifisch, oder aber bevorzugt gerichtet, über z.B. eine selektive Biotinylierung, oder gekoppelt über einen Spacer oder Linker erfolgen.

Dazu können die Bakteriophagenschwanzproteine mit niedermolekularen Substanzen z.B. Biotin verknüpft sein, um über diese niedermolekularen Substanzen an Polypeptide z. B. Streptavidin zu binden, die ihrerseits auf dem Träger immobilisiert wurden. Statt Biotin kann ferner der sogenannte Strep-Tag (Skerra, A. & Schmidt, T. G. M. Biomolecular Engineering 16 (1999), 79-86) verwendet werden, der eine kurze Aminosäuresequenz ist und an Streptavidin bindet. Ferner kann der His-Tag verwendet werden, der über zweiwertige Ionen (Zink oder Nickel) oder einen für ihn spezifischen Antikörper (Qiagen GmbH, Hilden) an ein Trägermaterial binden kann. Der Strep-Tag sowie der His-Tag wird vorzugsweise über DNA-Rekombinationstechnologie an die rekombinant hergestellten Bakteriophagenproteine gebunden. Diese Kopplung kann gerichtet, z.B. am N- oder C-Terminus oder an anderen Positionen im Bakteriophagenschwanzprotein erfolgen. Die gerichtete Kopplung erfolgt über eine geeignete, reaktive natürlicherweise bei Phagenproteinen nicht häufig oberflächenexponierte Aminosäure wie Cystein, das an geeigneter Stelle gezielt eingeführt wurde. Da Phagenschwanzproteine im Cytoplasma synthetisiert werden, ist nicht mit Disulfidbrücken zu rechnen. Vorzugsweise kann auch über andere Aminosäuren direkt, oder wie auch bei Cystein über einen "Spacer" oder "CrossLinker" (monofunktionell oder bifunktionell) indirekt gekoppelt werden.

Bei der Cysteinkopplung sind alle bifunktionellen Crosslinker mit NH- und SH-reaktiven Gruppen, mit und ohne Zwischenspacer, z.B. 11-Maleimidoundecanoic acid sulfo-NHS oder Succinimidyl-4-[N-maleimidomethyl]-cyclohexane-1-carboxy-[6-amido]caproate möglich. Sofern keine Spacer vorhanden sind, können 8-12 C-Atom-Spacer mit endständiger NH-Gruppe eingefügt werden. Vorzugsweise erfolgt die Cysteinkopplung über eine spezifische Biotinylierung des Cysteins durch z.B. EZ-Link-PEO-Maleimide activated Biotin (Pierce).

Zweiwertige Ionen z.B. Ca²⁺ oder Mg²⁺ sind für eine Bindung von Endotoxinen an Bakteriophagenschwanzproteine wie p12 von T4 oder die kurzen Schwanzfaserproteine der Phagen K3, T2, Ox2, RB32-33, AR1, PP01 oder RB69 wichtig. Durch Zugabe von geeigneten Chelatoren, wie z.B. EDTA oder EGTA, kann diese Bindung jedoch gelöst werden. Bevorzugt für die Bindung sind Ca²⁺-Konzentrationen im Bereich von etwa 0,1 µM bis etwa 100 mM, besonders bevorzugt im Bereich von etwa 0,1 µM bis etwa 10 mM, insbesondere bevorzugt im Bereich von etwa 0,1 µM bis etwa 1 mM und ferner insbesondere bevorzugt im Bereich von etwa 10 µM bis 1 mM. Ferner bevorzugt für die Bindung sind Mg²⁺-Konzentrationen im Bereich von etwa 0,1 µM bis etwa 10 mM, besonders bevorzugt im Bereich von etwa 0,1 µM bis etwa 1 mM, insbesondere bevorzugt im Bereich von etwa 10 µM bis 1 mM. Erniedrigt man die Konzentration zweiwertiger Ionen durch Zugabe von 1 mM EDTA unter 100 nM, so wird die Bindung von Endotoxin an p12 gelöst. Mg²⁺-Konzentrationen über 10 mM verschlechtern die Bindung von Endotoxin an p12, was sich in einer Erhöhung der Dissoziationskonstante bemerkbar macht. Ohne Zugabe von Mg²⁺ ergibt sich ein K_{d}-Wert von 50 nM und in einem Puffer mit 10 mM Mg²⁺ wurde ein K_{d}-Wert von 1 µM gemessen. Zink zeigte eine noch stärker hemmende Wirkung. 1 mM Zn erhöht den K_{d}-Wert auf 10 µM. Eine Einstellung der Konzentration zweiwertiger oder anderer Ionen (z.B.: Cu²⁺, Al³⁺, Zn²⁺, Fe²⁺, Ca²⁺, Ba²⁺, Mg²⁺, Cd²⁺) auf einen für die Bindung optimalen Bereich kann durch Substanzen, wie HEDTA, NTA bzw. allgemein Chelatoren/Puffer (ADA: N-[2-Acetamido]-2-iminodiacetic acid; 5-AMP: Adenosin-5'-Monophosphat; ADP: Adenosin-5'-Diphosphat; ATP: Adenosin-5'-Triphosphat; Bapta: 1,2-bis(2-Aminophenoxy)ethane-N,N,N',N'-tetraacetic acid; Citrat: Zitronensäure; EDTA: Ethylendiamintetraacetic acid; EGTA: Ethleneglycol-bis(β-aminoethyl Ether) N,N,N',N'-Tetraacetic acid; HEDTA: N-hydroxyethylethylenediaminetriacetic acid; NTA: Nitrilotiracetic acid; SO₄. Sulfat) erfolgen, die als Puffer für zweiwertige Ionen benutzt werden können.

Die erfindungsgemäßen Verfahren können daher ferner Waschschritte umfassen. Je nachdem, ob ein direkte oder indirekter Nachweis eine Abtrennung von Probe und Bakteriophagenschwanzprotein nötig macht, können Waschschritte eingebaut werden. Da Ca²⁺ oder andere Metallionen (z.B. Mg²⁺) essentiell für die Bindung sind, kann die Bindung von Endotoxin an z.B. p12 (z.B. die kurzen Schwanzfaserproteine der Phagen K3, T2, T4, Ox2, RB32-33, AR1, PP01 und RB69) durch geeignete Waschschritte gelöst werden. Je nach Ziel, ob Endotoxin auf dem Bakteriophagenschwanzprotein, z.B. p12 (z.B. die kurzen Schwanzfaserproteine der Phagen K3, T2, T4, Ox2, RB32-33, AR1, PP01 und RB69) gebunden bleiben soll, wird mit EDTA-freiem Puffer gewaschen, wenn die Bindung gelöst werden soll mit EDTA-haltigem Puffer, wobei die EDTA-Konzentrationen im Bereich von mindestens 0,05 mM bis mehr als 10 mM, vorzugsweise im Bereich von 2 mM bis 5 mM liegt.

Da ionische Wechselwirkungen grundsätzlich immer durch Veränderungen der Ionenstärke beeinflussbar sind, können auch Erhöhungen oder Erniedrigungen anderer Salze in Lösung, wie z.B. NaCl oder KCl, die Bindung von Endotoxin an die Bakteriophagenschwanzproteine beeinflussen.

Um die Bindung im Nachweisverfahren direkt oder indirekt sichtbar zu machen, kann auch das Protein molekularbiologisch oder biochemisch verändert werden, um die Messung zu ermöglichen, bzw. zu verbessern. Um eine Bindung von Endotoxin z.B. an p12 von T4 oder die kurzen Schwanzfaserproteine der Phagen K3, T2, T4, Ox2, RB32-33, AR1, PP01 oder RB69 oder die Endotoxin-bindenden Proteine von Bakteriophagen ohne Schwanz direkt sichtbar zu machen, kann ein molekularbiologischer Austausch von Tyrosinresten gegen Tryptophan durchgeführt werden. Für eine Reduktion des Signalhintergrundes kann es dabei nötig sein, die ursprünglich enthaltenen Tryptophane gegen Tyrosine auszutauschen. Um auch in proteinhaltigen Lösungen messen zu können, kann p12 von T4 oder die kurzen Schwanzfaserproteine der Phagen K3, T2, T4, Ox2, RB32-33, AR1, PP01 oder RB69 nach Tryptophan-Einführung zusätzlich chemisch modifiziert werden. Dabei werden Tryptophanreste durch Koshland-Reagenz (2-Hydroxy-5-nitrobenzylbromid) hinsichtlich ihrer spektrokopischen Eigenschaften verändert. Bei Verdrängungsexperimenten kann markiertes, z.B. fluoreszenzmarkiertes Endotoxin (z.B. Sigma) durch in der Probe befindliches Endotoxin z.B. von p12 von T4 oder die kurzen Schwanzfaserproteine der Phagen K3, T2, T4, Ox2, RB32-33, AR1, PP01 oder RB69 oder von z.B. PhiX 174 verdrängt und die Konzentration von freiem fluoreszierendem Endotoxin bestimmt werden.

Mit den erfindungsgemäßen Verfahren kann Endotoxin aus und in allen wässrigen Lösungen nachgewiesen werden. Diese Lösungen können Proteine, Plasmid-DNA, genomische DNA, RNA, Peptidoglykane, Polysaccharide, Protein-Nukleinsäurekomplexe wie z.B. Phagen oder Viren, Saccharide, Impfstoffe, Arzneimittel, Reaktionspuffer, Pufferlösungen allgemein, Medien, Dialysepuffer (Medizin), Salze, Blut, Blutbestandteile oder andere durch Endotoxin-Bindung verunreinigte Substanzen enthalten.

Ein weiterer Aspekt der Erfindung sind Bakteriophagenproteine, an die sogenannte Tags, z.B. der Strep- oder der His-Tag, vorzugsweise an den N- oder C-Terminus des Proteins, besonders bevorzugt an den C-Terminus, gekoppelt sind. Bevorzugt ist die Kopplung oder Verknüpfung der Tags mit den Bakteriophagenproteinen über DNA-Rekombinationstechnologie. Herstellung der Nukleinsäure, umfassend die Sequenz des Bakteriophagenproteins und des Tags und die Herstellung des Expressionsprodukts sind Stand der Technik und brauchen hier nicht gesondert erläutert zu werden. Ein weiterer Aspekt der Erfindung ist die Nukleinsäuresequenz, die ein Bakteriophagenprotein zusammen mit dem Strep- öder His-Tag codiert. Ein besonders bevorzugtes mit dem Strep- oder His-Tag modifiziertes Bakteriophagenprotein ist das p12-Protein vom Phagen T4, jedoch sind alle anderen Bakteriophagenproteine die an der Erkennnung und Bindung von Bakterien beteiligt oder dafür verantwortlich sind ebenfalls bevorzugt.

Für die erfindungsgemäßen Verfahren werden vorzugsweise Bakteriophagenproteine mit einem Tag verwendet, der ein oberflächenexponiertes Cystein zur spezifischen, gerichteten Biotinylierung aufweist, z.B. die Tags gemäß SEQ ID NO:5, 6 und 7. Ein Beispiel für ein p12 mit Tag ist die in SEQ ID NO:8 aufgeführte Aminosäuresequenz. Bevorzugt ist ein p12 mit einem Tag, insbesondere mit einem Tag mit einem oberflächenexponierten Cystein, insbesondere ein p12 mit dem Tag gemäß SEQ ID NO: 6 und 7. Diese gerichtete Biotinylierung kann zusätzlich durch einen geeigneten Spacer oder Linker vermittelt werden.

Die erfindungsgemäßen Verfahren bieten gegenüber den bisherigen Nachweisverfahren für Endotoxin Vorteile in der Performance entsprechender Anwendungen. Ferner ist die Herstellung von Antikörper gegen LPS-Herzoligosaccharide sehr schwierig, was entsprechende Verfahren auf Antikörper-Basis sehr teuer werden lässt.

Die vorliegende Erfindung betrifft ferner ein Endotoxin-Nachweis-Kit, umfassend die für das erfindungsgemäße Verfahren notwendigen Bestandteile. Das Kit umfasst insbesondere einen mit den hier beschriebenen Bakteriophagenschwanzproteinen beschichteten Träger, einen Behälter enthaltend ein Referenzendotoxin zur Erstellung und Messung einer Standardkurve, einen weiteren Behälter mit mindestens einem weiteren hier beschriebenen Bakteriophagenschwanzprotein, das gegebenenfalls für den Nachweis wie hier beschreiben modifiziert oder mit einem Aktiven Protein gekopplet sein kann oder einem Behälter mit Anti-Lipid A Antikörper zum Nachweis von Endotoxin.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.
Die im folgenden beschriebenen Experimente wurden mit p12-Proteinen von T4, T2 und K3 durchgeführt. Falls nicht explizit anderweitig angegeben, wurden die Ergebnisse nur für p 12 von T4 aufgeführt, da die Ergebnisse der drei Proteine aufgrund der hohen Homologie (Burda M.R., Hindenach I., Miller S., Biol. Chem. (2000) 381, 225-258) austauschbar sind (Riede I., Mol Gen Genet. 1987 Jan;206(1):110-115). Dies trifft ebenfalls auf die anderen in Figur 7 aufgeführten Proteine zu.

### Beispiel 1: Glasgefäße, Plastikgefäße und Puffer:

Für die Endotoxinentfernung wurden alle Glasgefäße durch Ausbacken bei 200°C (4h) entpyrogenisiert und ausschließlich pyrogenfreie Plastikmaterialen (z.B. Pipettenspitzen, Microtiterplatten) verwendet. Andere, nicht hitzebeständige Geräte oder Gefäße, wurden entweder mit 3% Wasserstoffperoxid behandelt oder mit 1% Natriumdeoxoycholat gewaschen. Anschließend wurde sie mit endotoxinfreiem Wasser gespült. Die Puffer wurden aus weitgehend endotoxinfreien Puffersubstanzen (Sigma) hergestellt und mit endotoxinfreiem Wasser angesetzt. Salze, wie z.B. NaCl, die auf 200°C erhitzt werden können, wurden ausgebacken (200°C, 4h). Für chromatographische Reinigungen verwendete Puffer wurden entgast und filtriert.

### Beispiel 2: Endotoxinnachweis mittels LAL-Test:

Endotoxin-Kontrollnachweise wurden mit einem chromogenen LAL-Test (Limulus-Amebocyte-Lysate Test, Charles-River Endosafe, Charleston, USA) entsprechend den Angaben des Herstellers durchgeführt. Zur Konzentrationsbestimmung wurden Endotoxin-Standards (Charles-River Endosafe, Charleston, USA) im Bereich von 0.005-50, bzw. 0.02-50 EU/ml eingesetzt. Die Absorptionsmessung bei 405 nm erfolgte in einem temperierbaren Mikrotiterplatten-Reader (Genios, Tecan GmbH).

### Beispiel 3: Western-Blot zum p12-Nachweis:

Der Nachweis von p12 im Überstand von mit Beads behandelten Proben bzw. in den Fraktionen der Affinitätschromatographie erfolgte durch Western Blots. Zum Teil wurden die Proteine vorher durch NaDOC/TCA-Fällung (Natriumdeoxycholat/Tetrachloracetat) aufkonzentriert. Die Proben wurden dazu auf 12%-igen SDS Gelen elektrophoretisch aufgetrennt und auf PVDF Membranen (Immobilon, Millipore) übertragen. Die Membranen wurden mit PBS 30 min gewaschen, mit 5% Milchpulver blockiert (1 h) und anschließend mit polyklonalem anti-p12 Antikörper inkubiert (1h, Verdünnung: 1: 1000). Nach Inkubation mit einem, mit alkalischer Phosphatase konjugierter Sekundärantikörper (Ziege-anti-Kaninchen IgG) erfolgte die Entwicklung der Proben mit BCIP/NBT (5-Brom-4-chloroindolylphosphat/Nitroblau-Tetrazoliumsalz).

### Beispiel 4: Endotoxin-Reinigung:

Die Reinigung von Endotoxin wurde nach der Vorschrift von Galanos, C., Lüderitz, O. & Westphal, O. 1969, Europ. J. Biochem. 9,245-249 durchgeführt.

### Beispiel 5: Spezifische Kopplung von p12 an immobilisierte Jodoacetylreste:

Um eine gerichtete Bindung von p12 an die Oberfläche zu erreichen wurde die Aminosäure Serin an Position 3 des Strep-Tags gemäß SEQ ID NO:5 durch Cystein wie in Beispiel 12 ersetzt und das Protein über Jodoacetylreste, die bevorzugt freie Sulfhydrylreste binden, immobilisiert. Das resultierende p12 wurde p12S3C genannt.

Es wurde ein 1 ml Sulfolink Coupling Gel (Pierce) gegossen, mit 6 ml 1% Natriumdeoxycholat gewaschen und mit 6 ml Kopplungspuffer (50 mM Tris, 150 mM NaCl, 5 mM EDTA, pH 8.5) equilibriert. Anschließend wurden 1 ml p12S3C (=N-StrepS3Cp12) (1-1.5 mg/ml in Kopplungspuffer) injiziert, die Säule 15 min leicht geschüttelt, weitere 30 min ohne Schütteln bei Raumtemperatur inkubiert, und nochmals 1 ml p12S3C injiziert und die Inkubationsschritte wiederholt. Diese Kopplung von p12S3C wurde insgesamt 4 mal wiederholt, und anschließend die Säule mit 6 ml Kopplungspuffer gewaschen. Die Durchläufe wurden gesammelt und die jeweilige p12S3C Konzentration durch Absorptionsmessung bei 280 nm bestimmt. Es wurden 2.2-2.8 mg p12S3C pro ml Gel gebunden. Anschließend wurden überzählige Jodoacetylreste durch Inkubation (45 min) mit 1 ml Cystein (50 mM in 50 mM Tris, 5 mM EDTA, pH 8.5) blockiert. Nach Waschen der Säule mit 16 ml 1M NaCl und 16 ml 20 mM Hepes, 150 mM NaCl pH 7.5 war die Säule fertig zum Gebrauch.

Die Fähigkeit dieses Gels Endotoxin aus Proteinlösungen zu entfernen, wurde mit BSA (2-4 mg/ml), Carbon Anhydrase (1-2 mg/ml) und Lysozym (3-4 mg/ml) getestet. BSA und Lysozym Lösungen wurden mit Endotoxin von E. coli O55:B5 (Charles-River Endosafe, Charleston, USA) oder E. coli HMS 174 gespickt (100-1000 EU/ml), während die Carbon Anhydrase nicht mit zusätzlichem Endotoxin versetzt wurde. Es wurde jeweils 0.5 ml Proteinlösung auf die Säule gegeben, 1 Stunde bei Raumtemperatur inkubiert und anschließend die Säule mit Puffer gewaschen. Die Proteine wurden fraktionsweise gesammelt und der Endotoxingehalt vor und nach der Säule mittels eines chromogenen LAL-Tests (Charles-River Endosafe, Charleston, USA) bestimmt. Außerdem wurde die Proteinwiederfindung durch Absorptionsmessungen bei 280 nm ermittelt. Die Endotoxine konnten aus allen 3 Proteinlösungen fast vollständig (93-99%) entfernt werden, wie in Fig. 2A gezeigt. Außerdem konnten die Proteine weitgehend von der Säule eluiert werden (80-99%, Fig. 2B). Die Säule wurde abschließend mit 5 mM EDTA, 20 mM Hepes, 150 mM NaCl, pH 7.5 regeneriert. Um Verunreinigungen der Proteinfraktionen nach dem Lauf über die Säule durch sich ablösendes p12 auszuschließen, wurden die Fraktionen mittels der Western Blot Technik auf p12 untersucht. Es konnte kein p12 in den Fraktionen nachgewiesen werden.

### Beispiel 6: Unspezifische Kopplung von p12 an NHS-aktiviertes Trägermaterial:

N-hydroxysuccinimid (NHS) wird aus Verbindungen durch primäre Aminoreste verdrängt und deshalb zum Koppeln von Proteinen an Oberflächen benutzt. NHS-aktivierte Sepharose Säulen (HiTrap NHS-activated HP, 1 ml, Amersham-Pharmacia-Biotech) wurden zunächst mit 6 ml eiskalter 1 mM Salzsäure gewaschen. Anschließend wurden bei Raumtemperatur 10-15 ml p12S3C (1.0-3.5 mg/ml) in 0.2 M NaHCO₃, 0.5 M NaCl, pH 8.3 zirkulär über die Säule gepumpt (Flussrate 0.8 ml/min). Nach 60 min wurde der Durchlauf fraktionsweise gesammelt und die Säule mit 6 ml Puffer gewaschen. Aus diesen Fraktionen wurde das NHS durch Entsalzen der Lösung über HiTrap-Desalting Säulchen (5 ml, Amersham-Pharmacia-Biotech) abgetrennt und anschließend die p12-Menge durch Absorptionsmessung bei 280 nm bestimmt. 20-25 mg p12S3C wurden an die Säule gebunden. Die Säule wurde nach der Kopplung entsprechend den Herstellerangaben wiederholt mit jeweils 6 ml Blockierungspuffer (0.5 M Ethanolamin, 0.5 M NaCl, pH 8.3) und Waschpuffer (0.1 M Acetat, 0.5 M NaCl, pH 4.0) gespült. Anschließend wurde die Säule mit 6 ml Gebrauchspuffer (20 mM Hepes, 150 mM NaCl, pH 7.5 oder 20 mM Tris, 150 mM NaCl, pH 8.5) equilibriert.

Die Endotoxinentfernung über diese Säule wurde mit Lysozymlösungen (3-4 mg/ml in 20 mM Hepes, 150 mM NaCl, pH 7.5 oder 20 mM Tris, 150 mM NaCl, pH 8.5) getestet. Die Lysozymlösungen wurden mit Endotoxin von E. coli HMS 174 gespickt (∼500 EU/ml). Es wurden 0.5 ml Proteinlösung auf die Säule gegeben, 1 Stunde bei Raumtemperatur inkubiert und anschließend die Säule mit Puffer gewaschen. Das Lysozym wurden fraktionsweise gesammelt und der Endotoxingehalt vor und nach der Säule mittels eines chromogenen LAL-Tests (Charles-River Endosafe, Charleston, USA) bestimmt. Außerdem wurde die Proteinwiederfindung durch Absorptionsmessungen bei 280 nm ermittelt. Die Endotoxine wurden zu 85-90% aus der Lösung entfernt, wie in Fig. 3A gezeigt und 85-90% des Lysozyms konnten durch Waschen mit Gebrauchspuffer wieder von der Säule eluiert werden (Fig. 3B). Die Säule wurde anschließend mit 6 ml 5 mM EDTA, 20 mM Hepes, 150 mM NaCl, pH 7.5 und 6 ml 1 M NaCl gewaschen. Um Verunreinigungen der Proteinfraktionen nach dem Lauf über die Säule durch sich ablösendes p12 auszuschließen, wurden die Fraktionen mittels der Western Blot Technik auf p12 untersucht. Es konnte kein p12 in den Fraktionen nachgewiesen werden.

### Beispiel 7: Gerichtete Kopplung von p12 an über Diaminoethan und N-Succinimidyl-iodoacetat (SIA) als Spacer an NHS-aktiviertes Trägermaterial-Säule.

Um eine gerichtete Bindung an das Chromatographie Trägermaterial zu erreichen wurde ein bifunktioneller Linker an NHS-aktivierte Oberfläche gebunden, der eine Kopplung von p12S3C über dessen freies Cystein und Jodoacetylreste des bifunktionalen Linkers ermöglicht.

NHS-aktivierte Sepharose Säulen (HiTrap NHS-activated HP, 1 ml, Amersham-Pharmacia-Biotech) wurden zunächst mit 6 ml eiskalter 1 mM Salzsäure gewaschen, danach 1 ml Ethylendiamin (10 mg/ml in 0.2 M NaHCO₃, 0.5 M NaCl, pH 8.3) injiziert und die Säule 30 min bei Raumtemperatur inkubiert. Nach Blockieren überzähliger NHS-Gruppen mit Ethanolamin (0.5 M Ethanolamin, 0.5 M NaCl, pH 8.3) und Waschen (0.1 M Acetat, 0.5 M NaCl, pH 4.0) der Säule wurde die Säule mit 6 ml Boratpuffer (50 mM Natriumborat, 150 mM NaCl, 5 mM EDTA, pH 8.3) equilibriert. Anschließend wurde 30 min lang10 ml N-Succinimidyl-iodoacetat (SIA, Pierce, 200 µl SIA-Stammlösung in 10 ml Boratpuffer; SIA-Stammlösung: 1.4 mg SIA in 1 ml DMSO) zirkulär über die Säule gespült. Die Säule wurde danach mit 6 ml Boratpuffer gewaschen und 1 Stunde lang p12S3C (1 mg/ml, 50 ml in Boratpuffer) über die Säule gespült. Überschüssige Iodoacetylreste wurden mit 1 ml Cysteinlösung (5mM Cystein in Boratpuffer, 15 min bei Raumtemperatur inkubieren) abgesättigt, bevor die Säule mit den Gebrauchspuffern (20 mM Hepes, 150 mM NaCl, pH 7.5 oder 50 mM Tris, 150 mM NaCl, pH 8.5) equilibriert wurden. Die Kopplungsreaktionen mit SIA wurden im Dunkeln durchgeführt.

Die Endotoxinentfernung über diese Säule wurde mit Lysozymlösungen (3-4 mg/ml in 20 mM Hepes, 150 mM NaCl, pH 7.5 oder 20 mM Tris, 150 mM NaCl, pH 8.5) getestet. Die Lysozymlösungen wurden mit Endotoxin von E. coli HMS 174 gespickt (-500 EU/ml). Es wurde 0.5 ml Proteinlösung auf die Säule gegeben, 1 Stunde bei Raumtemperatur inkubiert und anschließend die Säule mit Puffer gewaschen. Das Lysozym wurde fraktionsweiße gesammelt und der Endotoxingehalt vor und nach der Säule mittels eines chromogenen LAL-Tests (Charles-River Endosafe, Charleston, USA) bestimmt. Außerdem wurde die Proteinwiederfindung durch Absorptionsmessungen bei 280 nm ermittelt. Die Endotoxine wurden zu 90% aus der Lösung entfernt und 75-85% des Lysozyms konnten durch Waschen mit Gebrauchspuffer wieder von der Säule eluiert werden. Die Säule wurde anschließend mit 6 ml 5 mM EDTA, 20 mM Hepes, 150 mM NaCl, pH 7.5 und 6 ml 1 M NaCl gewaschen. Um Verunreinigungen der Proteinfraktionen nach dem Lauf über die Säule durch sich ablösendes p12 auszuschließen, wurden die Fraktionen mittels der Western Blot Technik auf p12 untersucht. Es konnte kein p12 in den Fraktionen nachgewiesen werden.

### Beispiel 8: Entfernung von Endotoxin aus einer BSA-Lösung im Durchflussverfahren

Hi-Trap-NHS aktivierte Sepharose (Amersham Biosciences, Uppsala, Schweden) wurde nach Vorschrift des Herstellers unspezifisch über primäre Aminogruppen mit p12 gekoppelt. Dabei wurden 8 mg p12 pro ml Gelmaterial kovalent immobilisiert. Die so erhaltene 1 ml Chromatographiesäule wurde mit einer Flussrate von 1 ml/min mit 10 ml Puffer A (20 mM HEPES pH 7.5, 150 mM NaCl, 0.1 mM CaC12) äquilibriert. Im Anschluß wurden 4 ml einer BSA Lösung (11.5mg BSA (Carl Roth GmbH, Deutschland) / ml Puffer A) aufgetragen (Injektion: I) und der Durchlauf (E) in 2,5 ml Fraktionen gesammelt. Die Säule wurde anschließend mit 15 ml Puffer A gewaschen und das an die Säule gebundene Endotoxin wurde mit 7 ml Puffer B (20 mM HEPES pH 7.5, 150 mM NaCl, 2 mM EDTA) eluiert. Bei Waschen und Elution wurden jeweils 2 ml Fraktionen gesammelt. Nach jedem Experiment wurde die Säule mit 20 ml Puffer C (20 mM HEPES pH 7.5, 150 mM NaCl, 2 mM EDTA, 0.1 % Natriumdesoxycholat) regeneriert. Die Endotoxin-Konzentration wurde durch einen chromogenen Limulus Amebocyte Lysate (LAL) Test (Charles-River Endosafe, Charleston, USA), nach Vorschrift des Herstellers bestimmt. Die Bestimmung der Proteinkonzentration erfolgte durch Messung der UV-Absorption. Die Endotoxin-Entfemungseffizienz betrug zwischen 95-99% und der Proteinverlust betrug etwa 6-10 %.

### Beispiel 9: Entfernung geringer Endotoxinmengen aus Puffer mittels unspezifisch gekoppeltem

p12. 20 ml NHS-aktivierte Sepharose 4 FastFlow (Amersham Biosciences) wurden zunächst mit eiskalter Salzsäure gewaschen und anschließend mit 292 mg p12 (7 mg/ml in 25 mM Citrat pH 7.0) 4 Stunden unter schütteln bei Raumtemperatur inkubiert. Anschließend wurde die Sepharose mit 7 x 80 ml 5 mM Citrat pH 2.0 gewaschen und jeweils 1 ml der Waschfraktionen gegen 5 mM Citrat pH 2.0 dialysiert. Diese Dialysate wurden benutzt, um das überschüssige p12 in den Waschfraktionen mittels Absorptionsmessung bei 280 nm zu quantifizieren. Es wurde eine Beladungsdichte von 8.7 mg p12 pro 1ml Sepharose bestimmt. Nicht abreagierte NHS-Reste wurden durch 12 h Inkubation der Sepharose mit 1M Tris pH 8.0 abgesättigt. Mit diesem Säulenmaterial wurden Säulen mit 2 ml Volumen gegossen und diese bei 4°C in 20% Ethanol bis zum Gebrauch gelagert.
In 3 Parallelversuchen wurde jeweils 4 ml Endotoxin Lösung (S) auf eine Säule aufgetragen. Die Endotoxin Lösung bestand aus Endotoxin von E. coli O55 :B5 (Charles-River Endosafe, Charleston, USA) in Equilibrationspuffer (20 mM Hepes, 150 mM NaCl, 0.1 mM CaCl₂, pH 7.5). Die Endotoxin Konzentration dieser Lösung lag bei 4.6 EU/ml.
Die Säulen wurde zunächst mit 12 ml Regenerationspuffer (20 mM Hepes, 150 mM NaCl, 2 mM EDTA, pH 7.5) und anschließend mit 12 ml Equilibrationspuffer gespült. Anschließend wurde nochmals Equlilibrationspuffer auf die Säule gegeben und 1ml fraktioniert.
Die Endotoxin Lösung wurden auf die Säulen aufgetragen (I) und Fraktionen von 5 ml und 2 ml gesammelt. Anschließend wurde die Säule mit 4 ml Regenerationspuffer (B) regeneriert. In den Durchlauffraktionen konnte kein Endotoxin detektiert werden, d.h. die Endotoxin Verunreinigungen konnten in allen drei Experimenten vollständig entfernt werden. Dieses Beispiel zeigt deutlich, dass auch bei geringen Endotoxinmengen eine Bindung erfolgt, was einem Nachweis geringer Endotoxinmengen entspricht.

### Beispiel 10: unspezifische Kopplung von biotinyliertem p12 an magnetische Streptavidin-Beads.

p12 (3 mg/ml in PBS, 0.05% Tween20) wurde mit Sulfo-NHS-LC-LC-Biotin (Pierce), im Verhältnis 1:10 bis 1:20 eine Stunde bei RT inkubiert und anschließend gegen Puffer (z.B. PBS oder 20 mM Hepes, 150 mM NaCl, 5 mM EDTA, pH 7.5) dialysiert. NHS-aktiviertes Biotin bindet dabei an primäre Aminoreste von p12. Anschließend wurden zu 1ml Streptavidin Beads (MagPrep Streptavidin Beads, Merck) 50 µl biotinyliertes p12 (1 mg/ml) gegeben, 2h bei Raumtemperatur geschüttelt und anschließend überschüssiges p12 durch viermaliges Waschen mit 1.5 ml 20 mM Tris, 10 mM EDTA, pH 7.5 entfernt.

Die Endotoxinentfernung wurde mit Puffer (20 mM Hepes, 150 mM NaCl, pH 7.5) und Proteinlösungen (0.1 mg/ml BSA, 0.1 mg/ml Lysozym, 0.1 mg/ml Carbon Anhydrase in 20 mM Hepes, 150 mM NaCl, pH 7.5) getestet. Der Puffer sowie die BSA- und Lysozym-Lösung wurde mit 5 EU/ml (Endotoxin aus E. coli O55:B5, Charles-River Endosafe, Charleston, USA) gespickt. Die Carbon Anhydrase Lösung enthielt etwa 1 EU/ml. Zu 200 µl Puffer bzw. Proteinlösung wurden 25 µl magnetische Beads mit immobilisiertem p12 gegeben, durch auf- und abpipettieren vermischt und 30 min bei Raumtemperatur inkubiert. Die Beads wurden mit Hilfe eines Magneten aus der Lösung entfernt, der Überstand abpipettiert. Der Endotoxingehalt von unbehandelten Proben und mit Beads inkubierten Proben wurde anschließend mit dem LAL-Test bestimmt und die Proteinwiederfindung durch Absorptionsmessung bei 280 nm bestimmt. Aus Puffer ließ sich das Endotoxin praktisch vollständig entfernen (99.9 % Endotoxinentfernung) und auch aus der Proteinlösung wurde das Endotoxin um 70-92% abgereichert. Die Proteinwiederfindung lag zwischen 57% und 99% (BSA: 87 %,Carbon Anhydrase: 99%, Lysozym: 57 %; Fig. 4B).

### Beispiel 11: unspezifische Kopplung von biotinyliertem p12 an immobilisiertes Streptavidin.

P12 (3 mg/ml in PBS, 0.05% Tween20) wurde mit Sulfo-NHS-LC-LC-Biotin (Pierce), im Verhältnis 1:10 bis 1:20 eine Stunde bei RT inkubiert und anschließend gegen Puffer (z.B. PBS oder 20 mM Hepes, 150 mM NaCl 5 mM EDTA, pH 7.5) dialysiert. NHS-aktiviertes Biotin bindet dabei an primäre Aminoreste von p12. Das biotinylierte p12 wird anschließend 1 h bei Raumtemperatur mit Streptavidin beladenen Chromatographiematerial (ImmunoPure immobilized Streptavidin: 6% quervernetzte Agarose Beads) inkubiert und überschüssiges p12 durch Waschen mit PBS entfernt.

Die Endotoxinentfernung wurde mit Puffer (20 mM Tris, 150 mM NaCl, pH 8.0) und BSA (0.5 mg/ml in 20 mM Tris, 150 mM NaCl, pH 8.0) getestet. Je 1 ml Puffer bzw. BSA-Lösung wurden mit 10 EU/ml gespickt, 50 µl p12-Agarose zugegeben, 1 Stunde bei Raumtemperatur geschüttelt. Die p12 Agarose wurde anschließend abzentrifugiert und die Endotoxin- und Proteinkonzentration im Überstand gemessen. Aus dem Puffer konnten 99% und aus der BSA-Lösung 86 % Endotoxin entfernt werden. BSA konnte zu 90 % wiedergefunden werden.

### Beispiel 12: Untersuchungen über die p12-Endotoxin Bindung mittels Oberflächen-Plasmon-Resonanz-Messungen

Die Bindung von p12 an Endotoxin oder an Bakterien, über die Lipopolysaccharide in der äußeren Zellmembran, wurde mittels Oberflächen-Plasmon-Resonanz Messungen untersucht (Biacore J). Um die Dissoziationskonstante (K_{d}) zu ermittelt, wurde Endotoxin von E. coli O55:B5 (Sigma) auf einem hydrophoben HPA-Chip entsprechend der Anleitung des Herstellers immobilisiert und p12 in verschiedenen Konzentrationen injiziert (Fig. 2A). Die Bindung wird in relativen "Response Units" (RU) gemessen die Gleichgewichtswerte gegen die dazugehörigen p12-Konzentrationen aufgetragen (Fig. 2B). Durch Anpassen der Langmuirschen Adsorptionsisotherme (RU = (RUₘₐₓ*[p12])/([p12]+K_{d})) an diese Daten wurde der K_{d}-Wert ermittelt (Tabelle 1). Für die Messungen wurden endotoxinfreie Puffer verwendet. Für pH-Werte zwischen 6 und 10 wurden K_{d}-Werte im Bereich von 10⁻⁷ bis 10⁻⁹ M ermittelt (Tabelle 1). Die Bindung wurde durch Injektion von 1mM oder 5 mM EDTA wiederaufgehoben und der Chip regeneriert.

**Tabelle 1: Dissoziationskonstanten von Endotoxin an p 12 in Abhängigkeit von dem pH-Wert der Lösung.**

| **pH** | **Kd** |
|---|---|
| 6,00 | 3,09E-07 |
| 7,50 | 6,85E-08 |
| 8,00 | 5,86E-08 |
| 8,50 | 7,86E-08 |
| 9,00 | 3,29E-08 |
| 10,00 | 1,55E-07 |

Um die Bindung von Bakterien an p12 zu untersuchen, wurde biotinyliertes p12 auf Streptavidin-Chips immobilisiert und verschiedene E. coli Stämme injiziert. Die Bakterien wurden für die Messungen in PBS aufgenommen. Es wurden E. coli Stämme verwendet, die Lipopolysaccharide mit unterschiedlichen Polysaccharid-Anteilen besitzen. Der Polysaccharidteil besteht aus einer "Herz"-Region, die mit dem Lipid A verknüpft ist und dem sogenannten O-Antigen. Das O-Antigen variert sehr stark zwischen verschiedenen Bakterienarten und auch Bakterienstämmen, während die "Herz"-Region stark konserviert ist. Stämme, die die "Herz"-Region und O-Antigen (z.B. E. coli), sowie Stämme die eine vollständige "Herz"-Region (E. coli D21) besitzen wurden von p12 gebunden, während Stämme mit einem stark verkürzter "Herz"-Region (z.B. E. coli D21f2) nicht mehr von p12 erkannt wurden (Fig. 2C). Die Bindung konnte durch EDTA (5 mM) wieder gelöst und der Chip regeneriert werden.

### Beispiel 13: rekombinante p12-Konstrukte

1. Konstruktion von p 12 mit N-terminalem Strep-Tag (N-Strep-p12): Mittels PCR wurde an das 5'-Ende des T4p12-Gens die Nukleotidsequenz für den Strep-Tag (US patent 5,506,121) eingeführt. Hierfür wurde für das 5'-Ende des p12-Gens ein Primer konstruiert (5'-GAA GGA ACT AGT CAT ATG *GCT AGC TGG AGC CAC CCG CAG TTC GAA AAA GGC GCC* AGT AAT AAT ACA TAT CAA CAC GTT-3' (SEQ ID NO:1), der die Nukleotidsequenz des Strep-Tags an seinem 5'-Ende beinhaltet (kursiv in der Sequenz) und eine Restriktionsschnittstelle (*Nde*I, unterstrichen in der Sequenz) derart besitzt, dass das Gen im richtigen Leseraster in das Expressionsplasmid eingesetzt werden kann. Für das 3'-Ende des p12-Gens wurde ein Primer konstruiert, der hinter dem p12-Gen eine *Bam*H I Restriktionsschnittstelle (kursiv in der Sequenz) einführt (5'-ACG CGC AAA GCT TGT CGA CGG ATC CTA TCA TTC TTT TAC CTT AAT TAT GTA GTT-3'), (SEQ ID NO:2). Die PCR wurde mit 40 Cyclen (1 min 95°C, 1 min 45°C und 1 min 72°C) durchgeführt. Der PCR-Ansatz wurde mit den Restriktionsendonukleasen *Nde*I und *Bam*HI geschnitten und das gewünschte Fragment nach Größenfraktionierung über ein Agarosegel und Elution aus dem Gel in die *Nde*I und *Bam*HI site des Expressionsplasmids pET21a eingesetzt. Die Sequenz des N-Strep-p12-Gens wurde über DNA-Sequenzierung auf seine Richtigkeit hin überprüft. Die weiteren Schritte zum Plasmid pNS-T4p12p57 wurden wie von Burda, M.R. & Miller, S. (Eur J Biochem. 1999 265 (2), 771-778) für T4p12p57 beschrieben durchgeführt. Das Plasmid pNS-T4p12p57 wurde dann in den Expressionsstamm BL21 (DE3) transformiert.
2. Einfügen eines N-terminalen Cysteinrests in N-Strep-p12 (N-Strep-S3C-p12 und N-Strep-S14C-p12): Die Einfügung eines N-terminalen Cysteinrestes wurde wie unter 1. beschrieben durchgeführt, wobei dafür zwei neue Primer für das 5'-Ende konstruiert wurden. Für das N-Strep-S3C-p12 wurde der Primer 5'-GAA GGA ACT AGT CAT ATG *GCT **TGT** TGG AGC CAC CCG CAG TTC GAA AAA GGC GCC* AGT AAT AAT ACA TAT CAA CAC GTT-3' (SEQ ID NO:3), für das N-Strep-S14C-p12 wurde der Primer 5'-GAA GGA ACT AGT CAT ATG *GCT AGC TGG AGC CAC CCG CAG TTC GAA AAA GGC GCC* TGT AAT AAT ACA TAT CAA CAC GTT-3' (SEQ ID NO:4)verwendet.
3. Reinigung von N-Strep-p12 Protein: Der *E. coli* Stamm BL21(DE3) mit dem Plasmid pNS-T4p12p57 wurde in 2 1 Schüttelkulturen (LB-Medium mit Ampicillin 100 µg/ml) bis zu einer OD600 von 0.5-0.7 bei 37°C gezogen und die Expression des N-Strep-p12-Proteins wurde durch Zugabe von 1mM IPTG (Isopropyl-β-thio-galactopyranoside) induziert. Nach Inkubation bei 37°C für 4h wurden die Zellen abgeerntet. Geerntete Zellen aus 10 1 Kultur wurden in 50 ml Natriumphosphat, 20 mM pH 7.2, 2 mM MgSO4, 0.1 M NaCl aufgenommen, durch dreimalige French-Press-Behandlung (20.000 psi) aufgebrochen und anschließend 30 min bei 15.000 rpm (SS34) abzentrifugiert. Nach zweimaligem Waschen im gleichen Puffer wurde das N-Strep-p12 Protein aus dem Pellet das Pellet 3x mit durch Rühren für 30 min in 40 mM TrisHCl pH 8.0, 10 mM EDTA extrahiert, der Ansatz für 30 min bei 15.000 rpm (SS34) zentrifugiert und das abgelöste NS-p12 im Überstand bei 4°C gelagert. Die Extraktion wurde zweimal wiederholt. und die vereinigten Überstände wurden auf eine Streptactin-Affinitätssäule (15 ml), äquilibriert mit Puffer "W" (100 mM TrisHCl pH 8, 1 mM EDTA, 150 mM NaCl), aufgetragen (IBA GmbH, Göttingen). Nach Waschen mit 5 Säulenvolumina Puffer "W" wurde mit 3 Volumina Puffer "W" mit 2.5 mM Desthiobiotin in Puffer "W" eluiert. Nach mehrmaliger Dialyse gegen Puffer "W" und Aufkonzentration wurde über SDS-PAGE und UV-Spektroskopie (Burda et.al. 1999) die Konzentration und Reinheit von N-Strep-T4p 12 ermittelt. Aus 10 Liter Kultur wurden so ca. 100 mg N-Strep-T4p12 gereinigt.

| Name | Sequenz des Tag | |
|---|---|---|
| Nstrep-p12 | MASWSHPQFEKGAS | SEQ ID NO: 5 |
| Nstrep-p12-S3C | MACWSHPQFEKGAS | SEQ ID NO: 6 |
| Nstrep-p12-S14C | MASWSHPQFEKGAC | SEQ ID NO: 7 |

### Beispiel 14: Nachweis von LPS über die Bindung von p12 an immobilisiertes LPS.

Es wurden mit Polymyxin B Sepharose (Detoxi-Gel, Pierce) 4 Säulen mit je 0.5 ml Volumen gegossen. Die Säulen wurden mit je 3 ml Natriumphosphat Puffer (20 mM Natriumphosphat, pH 12.0) und je 3 ml Regenerationspuffer (20 mM Hepes, 150 mM NaCl, 2 mM EDTA, pH 7.5) gewaschen. Anschließend wurden auf zwei dieser Säulen je 1 ml LPS von E. coli O55:B5 aufgetragen (0.1 mg/ml in Hepes Puffer, 10⁶ EU/ml). Die zwei anderen Säulen wurden mit je 1 ml Regenerationspuffer gespült. Danach wurden alle Säulen mit je 3 ml Equilibrationspuffer (20 mM Hepes, 150 mM NaCl, 0.1 mM CaCl₂, pH 7.5) gewaschen und anschließend nochmals 1 ml dieses Puffers aufgetragen und als Fraktion 1 aufgefangen. Anschließend wurden 0.5 ml einer Lösung mit dem Bakteriophagenschwanzprotein p12 (0.844 mg/ml in 20 mM Hepes, 150 mM NaCl, 0.1 mM CaCl₂) auf die Säulen aufgetragen und mit 2.5 ml Equilibrationspuffer und 2 ml Regenerationspuffer gewaschen. Der Durchlauf wurde in Fraktionen von dreimal 1 ml und einmal 2 ml aufgefangen und die Konzentration des Bakteriophagenschwanzproteins p12 in diesen Fraktionen mittels Absorptionsmessung bei 280 nm bestimmt (Figur 4). Das Bakteriophagenschwanzprotein p12 wurde zum großen Teil an die Säulen gebunden, die vorher mit LPS behandelt worden waren, und konnte durch Zugabe von Regenerationspuffer von diesen Säulen gelöst werden. Im Gegensatz dazu lief es ohne Verzögerung durch die Säulen, die nicht mit LPS behandelt worden waren.

### Beispiel 15: Nachweis der Bindung von Endotoxin-Polysaccharid an die T4p12 Mutante W359 Y W283Y.

In der verwendeten T4p12 Mutante ist an den Positionen 359 und 283 die Aminosäure Tryptophan gegen Tyrosin ersetzt. Für die Fluoreszenzuntersuchungen wurde ein Polysaccharid (MW = 2 kDa), das aus dem Endotoxin von *Salmonella typhimurium* stammt, verwendet. Sowohl die p12 Mutante (40 oder 200 µg/ml) als auch das Endotoxin-Polysaccharid waren in 5 mM Citrat pH 2 gelöst worden. Die Fluoreszenz im Bereich von 305-450 nm wurde durch Anregung bei 295 nm. Es wurden 120 µl eine Lösung mit der p12 Mutante W359 Y W283Y in einer Fluoreszenzküvette vorgelegt die Fluoreszenz gemessen und anschließend schrittweise Polysaccharid zugegeben(Endkonzentration: 0.5 - 120000 nM) und nach mischen der Probe erneut die Fluoreszenz gemessen. In Kontrollversuchen wurde derselbe Versuch ohne die p12 Mutante durchgeführt und die Messkurven gegen diese Daten korrigiert.
Die Bindung von Endotoxin oder einem Endotoxin-Polysaccharid reduziert die Fluoreszenz dieser Mutante (Figur 5). Dies kann für den Nachweis von Endotoxin eingesetzt werden.

### Beispiel 16: Immobilisierung von Lipopolysaccharid über T4p12 und Nachweis der Bindung

über einen anti-Lipid A Antikörpers. Zum Nachweis sollen Lipopolysaccharide zunächst aus einer Probenlösung an eine Oberfläche gebunden werden und anschließend diese immobilisierten Lipopolysaccharide mit Hilfe eines zweiten Proteins, das an Lipopolysaccharide bindet, nachgewiesen werden. Um die Machbarkeit dieser Nachweismethode darzustellen, wurde solcher ein "Sandwich" auf der Oberfläche eines Biacore Chips konstruiert und die Bindung von Lipopolysaccharid und eines Lipid A Antikörpers mittels Oberflächen-Plasmon-Resonanzmessung verfolgt.
Dazu wurde zunächst T4p12 an die Oberfläche eines CM-5 Chips (Biacore) kovalent gekoppelt. Die Carboxylreste der Oberfläche wurden mit einer Mischung aus EDC (N-(3-dimethylamionpropyl)carbodiimide hydrochloride, 75 mg/ml) und NHS (N-hydroxysuccinimide, 11.5 mg/ml) aktiviert und anschließend T4p12 injiziert, dessen primäre Aminoreste mit der aktivierten Oberfläche reagierten. Die Oberfläche einer zweiten Referenzzelle blieb unbehandelt. Die Bindung von 100 µl Lipopolysaccharid (E. coli O55:B5, 0.1 mg/ml) an T4p12 konnte anhand der Zunahme des Resonanzsignals verfolgt werden (siehe Fig 6). Auch nach dem Ende der Injektion blieb das Resonanzsignal auf seinem Niveau und zeigt damit eine stabile Bindung an. Nachfolgende Injektionen eines anti-Lipid A Antikörpers (2 µg/ml, polyklonaler Antiköper gegen Lipid A aus Ziege von Accurate Chemical & Scientific Corporation, Produkt Nummer YVS6921) zeigten ebenfalls eine Zunahme des Resonanzsignals und damit eine Bindung des Antikörpers. Das Resonanzsignal konnte durch Injektion von EDTA (2 mM), das die Bindung von Lipopolysaccharid and T4p12 aufhebt, wieder auf das Ausgangsniveau gesenkt werden. Dies bedeutet, dass die Signalzunahme, die durch die Injektion des Antikörpers induziert wurde, durch die Bindung an Lipopolysaccharid erfolgte.
Die Experimente wurden in folgendem Puffer durchgeführt: 20 mM Hepes, 150 mM NaCl, 0.1 mM CaCl₂, pH 7.5.

Beispiel 17: Immobilisierung von Lipopolysaccharid über T2p12 und Nachweis der Bindung über einen anti-Lipid A Antikörpers. T2p12 wurde analog zu T4p12 kloniert, exprimiert und gereinigt. Zum Nachweis werden Lipopolysaccharide zunächst aus einer Probenlösung an eine Oberfläche gebunden und anschließend diese immobilisierten Lipopolysaccharide mit Hilfe eines zweiten Proteins, das an Lipopolysaccharide bindet, nachgewiesen. Um die Machbarkeit dieser Nachweismethode darzustellen, wurde solcher ein "Sandwich" auf der Oberfläche eines Biacore Chips konstruiert und die Bindung von Lipopolysaccharid und eines Lipid A Antikörpers mittels Oberflächen-Plasmon-Resonanzmessung verfolgt.
Dazu wurde zunächst T2p12 an die Oberfläche eines CM-5 Chips (Biacore) kovalent gekoppelt. Die Carboxylreste der Oberfläche wurden mit einer Mischung aus EDC (N-(3-dimethylamionpropyl)carbodiimide hydrochloride, 75 mg/ml) und NHS (N-hydroxysuccinimide, 11.5 mg/ml) aktiviert und anschließend T2p12 injiziert, dessen primäre Aminoreste mit der aktivierten Oberfläche reagierten. Die Oberfläche einer zweiten Referenzzelle blieb unbehandelt. Die Bindung von 100 µl Lipopolysaccharid (E. coli O55:B5, 0.1 mg/ml) an T2p12 konnte anhand der Zunahme des Resonanzsignals verfolgt werden. Auch nach dem Ende der Injektion blieb das Resonanzsignal auf seinem Niveau und zeigt damit eine stabile Bindung an. Nachfolgende Injektionen eines anti-Lipid A Antikörpers (2.1 µg/ml, polyklonaler Antiköper gegen Lipid A aus Ziege von Accurate Chemical & Scientific Corporation, Produkt Nummer YVS6921) zeigten ebenfalls eine Zunahme des Resonanzsignals und damit eine Bindung des Antikörpers. Das Resonanzsignal konnte durch Injektion von EDTA (2 mM), das die Bindung von Lipopolysaccharid and T2p12 aufhebt, wieder auf das Ausgangsniveau gesenkt werden. Dies bedeutet, dass die Signalzunahme, die durch die Injektion des Antikörpers induziert wurde, durch die Bindung an Lipopolysaccharid erfolgte.

Die Experimente wurden in folgendem Puffer durchgeführt: 20 mM Hepes, 150 mM NaCl, 0.1 mM CaCl₂, pH 7.5.

Beispiel 18: Immobilisierung von Lipopolysaccharid über RB69p12 und Nachweis der Bindung über einen anti-Lipid A Antikörpers. RB69p12 wurde analog zu T4p12 kloniert, exprimiert und gereinigt. Zum Nachweis werden Lipopolysaccharide zunächst aus einer Probenlösung an eine Oberfläche gebunden und anschließend diese immobilisierten Lipopolysaccharide mit Hilfe eines zweiten Proteins, das an Lipopolysaccharide bindet, nachgewiesen. Um die Machbarkeit dieser Nachweismethode darzustellen, wurde solcher ein "Sandwich" auf der Oberfläche eines Biacore Chips konstruiert und die Bindung von Lipopolysaccharid und eines Lipid A Antikörpers mittels Oberflächen-Plasmon-Resonanzmessung verfolgt.
Dazu wurde zunächst RB69p12 an die Oberfläche eines CM-5 Chips (Biacore) kovalent gekoppelt. Die Carboxylreste der Oberfläche wurden mit einer Mischung aus EDC (N-(3-dimethylamionpropyl)carbodiimide hydrochloride, 75 mg/ml) und NHS (N-hydroxysuccinimide, 11.5 mg/ml) aktiviert und anschließend RB69p12 injiziert, dessen primäre Aminoreste mit der aktivierten Oberfläche reagierten. Die Oberfläche einer zweiten Referenzzelle blieb unbehandelt. Die Bindung von 100 µl Lipopolysaccharid (E. coli O55:B5, 0.1 mg/ml) an RB69p12 konnte anhand der Zunahme des Resonanzsignals verfolgt werden. Auch nach dem Ende der Injektion blieb das Resonanzsignal auf seinem Niveau und zeigt damit eine stabile Bindung an. Nachfolgende Injektionen eines anti-Lipid A Antikörpers (2.1 µg/ml, polyklonaler Antiköper gegen Lipid A aus Ziege von Accurate Chemical & Scientific Corporation, Produkt Nummer YVS6921) zeigten ebenfalls eine Zunahme des Resonanzsignals und damit eine Bindung des Antikörpers. Das Resonanzsignal konnte durch Injektion von EDTA (2 mM), das die Bindung von Lipopolysaccharid and RB69p12 aufhebt, wieder auf das Ausgangsniveau gesenkt werden. Dies bedeutet, dass die Signalzunahme, die durch die Injektion des Antikörpers induziert wurde, durch die Bindung an Lipopolysaccharid erfolgte.
Die Experimente wurden in folgendem Puffer durchgeführt: 20 mM Hepes, 150 mM NaCl, 0.1 mM CaCl₂, pH 7.5.

## Patentansprüche

1. Verfahren zum Nachweis von Endotoxin, umfassend die Schritte:
a) In Kontakt bringen einer Probe enthaltend Endotoxine mit einer mit Endotoxinbindender Substanz beschichteten Oberfläche, anschließend
b) Inkubieren von Bakteriophagenschwanzproteinen oder Bakteriophagenkopfproteinen von Bakteriophagen mit Schwanz oder Bakteriophagenhüllproteinen von Bakteriophagen ohne Schwanz an die auf der Oberfläche immobilisierten Endotoxine, und
c) Nachweis der Bakteriophagenschwanzproteine oder Bakteriophagenkopfproteine von Bakteriophagen mit Schwanz oder Bakteriophagenhüllproteine von Bakteriophagen ohne Schwanz mittels spektroskopischer Verfahren, ELISA, chemischer oder enzymatischer Nachweisreaktion von Endotoxinen oder abgespaltenen Endotoxinkomponenten, oder mittels Kapazitätsmessung.

2. Verfahren nach Anspruch 1, ferner umfassend nach Schritt b) und vor Schritt c) den zusätzlichen Schritt
b') Abtrennen der gebundenen Bakteriophagenschwanzproteine oder Bakteriophagenkopfproteine von Bakteriophagen mit Schwanz oder der Bakteriophagenhüllproteine von Bakteriophagen ohne Schwanz vom Endotoxin.

3. Verfahren nach Anspruch 1, wobei die Endotoxin-bindende Substanz ein Bakteriophagenschwanzprotein oder Bakteriophagenkopfprotein von Bakteriophagen mit Schwanz oder ein Bakteriophagenhüllprotein von Bakteriophagen ohne Schwanz ist.

4. Verfahren nach einem der vorhergehenden Ansprüche, wobei das Bakteriophagenschwanzprotein ein Protein der kurzen Bakteriophagenschwanzfaser ist.

5. Verfahren nach Anspruch 4, wobei das Protein der kurzen Bakteriophagenschwanzfaser ausgewählt ist aus K3, T2, T4, Ox2, RB32-33, AR1, PP01 und RB69.

6. Verfahren nach Anspruch 4 oder 5, wobei das Bakteriophagenschwanzprotein eine Homologie auf Aminosäureebene von mindestens 60 % zum T4p12 Protein aufweist.

7. Verfahren nach einem der vorhergehenden Ansprüche, wobei die Bakteriophagenschwanzproteine oder Bakteriophagenkopfproteine von Bakteriophagen mit Schwanz oder die Bakteriophagenhüllproteine von Bakteriophagen ohne Schwanz modifiziert sind.

8. Verfahren nach einem der vorhergehenden Ansprüche, wobei die Bakteriophagenschwanzproteine oder Bakteriophagenkopfproteine von Bakteriophagen mit Schwanz oder die Bakteriophagenhüllproteine von Bakteriophagen ohne Schwanz mit enzymatisch aktiven Proteinen kovalent verbunden sind.

9. Verfahren nach einem der vorhergehenden Ansprüche, wobei das Bakteriophagenschwanzprotein oder Bakteriophagenkopfprotein von Bakteriophagen mit Schwanz oder das Bakteriophagenhüllprotein von Bakteriophagen ohne Schwanz einen Strep-Tag oder einen His-Tag aufweist.

10. Verfahren nach Anspruch 9, wobei der Tag eine Aminosäuresequenz gemäß SEQ ID NO. 5, 6 oder 7 aufweist.

11. Verfahren nach Anspruch 9 oder 10, wobei als Bakteriophagenschwanzprotein das p12-Protein des Phagen T4, K3, T2, Ox2, RB32-33, AR1, PP01 oder RB69 verwendet wird.

12. Verfahren nach einem der vorhergehenden Ansprüche, wobei die Ca²⁺-Konzentration in der Inkubation 0,1 µM bis 10 mM und/oder die Mg²⁺-Konzentration 0,1 µM bis 10 mM beträgt.

13. Verfahren nach einem der vorhergehenden Ansprüche, wobei markiertes Endotoxin aus der Bindung mit einem Bakteriophagenschwanzprotein oder Bakteriophagenkopfprotein von Bakteriophagen mit Schwanz oder einem Bakteriophagenhüllprotein von Bakteriophagen ohne Schwanz verdrängt wird und das markierte Endotoxin anschließend nachgewiesen wird.

14. Ein Endotoxin-Nachweis-Kit, umfassend einen mit einer Endotoxin-bindenden Substanz beschichteten Träger, einen Behälter enthaltend ein Referenzendotoxin zur Messung einer Standardkurve, und einen Behälter mit mindestens einem Bakteriophagenschwanzprotein oder Bakteriophagenkopfprotein von Bakteriophagen mit Schwanz oder einem Bakteriophagenhüllprotein von Bakteriophagen ohne Schwanz, oder einem Anti-Lipid A Antikörper.

## Claims

1. Method for detection of endotoxin, the method comprising the steps of:
a) contacting a sample containing endotoxins with a surface coated with an endotoxin binding substance, subsequently
b) incubating of bacteriophage tail proteins or bacteriophage head proteins of bacteriophages with tail or bacteriophage coat proteins of bacteriophages without tail with the endotoxins immobilised on the surface, and
c) detection of bacteriophage tail proteins or bacteriophage head proteins of bacteriophages with tail or bacteriophage coat proteins of bacteriophages without tail by means of spectroscopic methods, ELISA, chemical or enzymatic detection reaction of endotoxins or cleaved-off endotoxin components, or by means of capacitance measurements.

2. Method according to claim 1 further comprising after step b) and before step c) an additional step
b') separation of said bound bacteriophage tail proteins or bacteriophage head proteins of bacteriophages with tail or bacteriophage coat proteins of bacteriophages without tail from endotoxin.

3. Method according to claim 1, wherein the endotoxin binding substance is a bacteriophage tail protein or bacteriophage head protein of bacteriophages with tail or bacteriophage coat protein of bacteriophages without tail.

4. Method according to any one of the preceding claims, wherein the bacteriophage tail protein is a protein of the short bacteriophage tail fiber.

5. Method according to claim 4, wherein the protein of the short bacteriophage tail fiber is selected from K3, T2, T4, Ox2, RB32-33, AR1, PP01 and RB69.

6. Method according to claims 4 or 5, wherein the bacteriophage tail protein has a homology of at least 60 % to T4p 12 protein on the amino acid level.

7. Method according to any one of the preceding claims, wherein the bacteriophage tail proteins or bacteriophage head proteins of bacteriophages with tail or bacteriophage coat proteins of bacteriophages without tail are modified.

8. Method according to any one of the preceding claims, wherein the bacteriophage tail proteins or bacteriophage head proteins of bacteriophages with tail or bacteriophage coat proteins of bacteriophages without tail are covalently linked to enzymatically active proteins.

9. Method according to any one of the preceding claims, wherein the bacteriophage tail protein or bacteriophage head protein of bacteriophages with tail or bacteriophage coat protein of bacteriophages without tail comprises a strep-tag or a his-tag.

10. Method according to claim 9, wherein the tag comprises an amino acid sequence according to SEQ ID NOS 5, 6 or 7.

11. Method according to claim 9 or 10, wherein the p12-protein of phage T4, K3, T2, Ox2, RB32-33, AR1, PP01 or RB69 is used as bacteriophage tail protein.

12. Method according to any one of the preceding claims, wherein the Ca²⁺ concentration is in the incubation 0.1 µM to 10 mM and/or the Mg²⁺ concentration is 0.1 µM to 10 mM.

13. Method according to any one of the preceding claims, wherein marked endotoxin is displaced from the binding with a bacteriophage tail protein or bacteriophage head protein of bacteriophages with tail or bacteriophage coat protein of bacteriophages without tail and wherein the marked endotoxin is detected subsequently.

14. An endotoxin detection kit comprising a carrier coated with an endotoxin binding substance, a container containing a reference endotoxin for measurement of a standard curve, and a container with at least one bacteriophage tail protein or bacteriophage head protein of bacteriophages with tail or bacteriophage coat protein of bacteriophages without tail or an anti lipid A antibody.

## Revendications

1. Procédé de détection d'endotoxine, comprenant les étapes de :
a) mise en contact d'un échantillon contenant des endotoxines avec une surface enduite d'une substance liant l'endotoxine, suivie de
b) incubation de protéines de queue ou de tête de bactériophage à queue ou de protéines d'enveloppe de bactériophage sans queue sur les endotoxines immobilisées sur la surface, et
c) détection des protéines de queue ou de tête de bactériophage à queue ou des protéines d'enveloppe de bactériophage sans queue au moyen de procédés spectroscopiques, de la méthode ELISA, de la réaction de détection chimique ou enzymatique des endotoxines ou des composants d'endotoxine clivés, ou au moyen d'une mesure de capacité.

2. Procédé selon la revendication 1, comprenant en outre après l'étape b) et avant l'étape c) l'étape supplémentaire de
b') séparation des protéines de queue ou de tête de bactériophage à queue ou des protéines d'enveloppe de bactériophage sans queue liées et de l'endotoxine.

3. Procédé selon la revendication 1, dans lequel la substance liant l'endotoxine est une protéine de queue ou de tête de bactériophage à queue ou une protéine d'enveloppe de bactériophage sans queue.

4. Procédé selon l'une des revendications précédentes, dans lequel la protéine de queue de bactériophage est une protéine de fibre de queue de bactériophage courte.

5. Procédé selon la revendication 4, dans lequel la protéine de fibre de queue de bactériophage courte est choisie parmi K3, T2, T4, Ox2, RB32-33, AR1, PP01 et RB69.

6. Procédé selon la revendication 4 ou 5, dans lequel la protéine de queue de bactériophage présente une homologie au niveau des acides aminés d'au moins 60 % avec la protéine T4p 12.

7. Procédé selon l'une des revendications précédentes, dans lequel les protéines de queue ou de tête de bactériophage à queue ou les protéines d'enveloppe de bactériophage sans queue sont modifiées.

8. Procédé selon l'une des revendications précédentes, dans lequel les protéines de queue ou de tête de bactériophage à queue ou les protéines d'enveloppe de bactériophage sans queue sont liées de manière covalente avec des protéines enzymatiquement actives.

9. Procédé selon l'une des revendications précédentes, dans lequel la protéine de queue ou de tête de bactériophage à queue ou la protéine d'enveloppe de bactériophage sans queue présente un tag Strep ou un tag His.

10. Procédé selon la revendication 9, dans lequel le tag présente une séquence d'acides aminés conforme à la SEQ ID N°5, 6 ou 7.

11. Procédé selon la revendication 9 ou 10, dans lequel on utilise comme protéine de queue de bactériophage la protéine p12 du phage T4, K3, T2, Ox2, RB32-33, AR1, PP01 ou RB69.

12. Procédé selon l'une des revendications précédentes, dans lequel la concentration en Ca²⁺ dans l'incubation atteint 0,1 µM à 10 mM et/ou la concentration en Mg²⁺ atteint 0,1 µM à 10 mM.

13. Procédé selon l'une des revendications précédentes, dans lequel l'endotoxine marquée est libérée de la liaison avec une protéine de queue ou de tête de bactériophage à queue ou une protéine d'enveloppe de bactériophage sans queue et l'endotoxine marquée est ensuite détectée.

14. Kit de détection de l'endotoxine, comprenant un support enduit d'une substance liant l'endotoxine, un récipient contenant une endotoxine de référence pour mesurer une courbe standard, et un récipient avec au moins une protéine de queue ou de tête de bactériophage à queue ou une protéine d'enveloppe de bactériophage sans queue, ou un anticorps anti-lipide A.
